# FASCICULE DE BREVET EUROPEEN

(11) **EP 2 142 501 B1**
(45) Date de publication et mention de la délivrance du brevet: **08.10.2014**
(21) Numéro de dépôt: 08787880.7
(22) Date de dépôt: 31.03.2008
(51) Int. Cl.: C07D 207/34, C07D 487/22

(54) **PROCEDE DE PREPARATION DE DERIVES DE PORPHYRINE, TELLE QUE LA PROTOPORPHYRINE (IX) ET INTERMEDIAIRE DE SYNTHESE**
VERFAHREN ZUR HERSTELLUNG VON PORPHYRINDERIVATEN, Z.B. PROTOPORPHYRIN (IX), UND SYNTHESE-ZWISCHENPRODUKTEN
PROCESS FOR PREPARING PORPHYRIN DERIVATIVES, SUCH AS PROTOPORPHYRIN (IX) AND SYNTHESIS INTERMEDIATE

(30) Priorité: 30.03.2007 FR 0702334
(43) Date de publication de la demande: 13.01.2010
(73) Titulaire: Sanofi Pasteur, 69367 Lyon Cedex 07 (FR)
(72) Inventeur: MARTIN, Pierre, CH-4310 Rheinfelden (CH); MÜLLER, Markus, CH-4317 Wegenstetten (CH); SPIELVOGEL, Dirk, 79540 Lörach (DE); FLUBACHER, Dietmar, 79189 Bad Krozingen (DE); BOUDIER, Andreas, CH-4125 Riehen (CH)
(86) Numéro de dépôt international: PCT/FR2008/000440
(87) Numéro de publication internationale: WO 2008/142250

(56) Documents cités:
- B. FRYDMAN ET AL.: "Carbon-5 regiospecific synthesis of deuteroporphyrin IX" JOURNAL OF ORGANIC CHEMISTRY, vol. 47, 1982, pages 3059-3063, XP002460570
- G. W. KENNER: "Pyrroles and related compounds. Part XXXIII. Biosynthesis of protoporphyrin-IX from coproporphyrinogen-III" J. C. S. PERKIN I, 1974, pages 1188-1194, XP008086394 cité dans la demande
- Y. NAKAE ET AL: "The convenient screening method for using albumin for the tumor localizing of Ga-porphyrin complexes" JOURNAL OF PHOTOCHEMISTRY AND PHOTOBIOLOGY A: CHEMISTRY, vol. 172, 2005, pages 55-61, XP002460571 cité dans la demande

## Description

La présente invention concerne un nouveau procédé de préparation de dérivés de porphyrine, telle que la protoporphyrine (IX) ainsi que des intermédiaires pour la synthèse de ces composés.

Certaines porphyrines sont connues et utilisées pour leurs propriétés biologiques ou médicales. A titre d'exemple, on peut citer les porphyrines suivantes de formule : 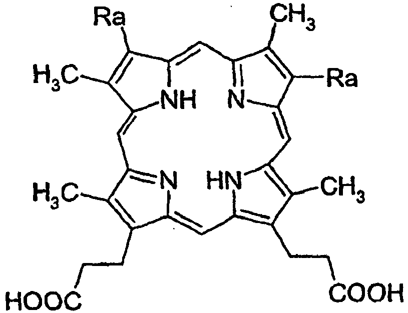 dans laquelle :
Ra = -CH=CH₂, alors nommée protoporphyrine IX,
Ra = -CH₂CH₃, alors nommée mesoporphyrine,
Ra = -CH(OH)CH₃, alors nommée hématoporphyrine,
Ra = H, alors nommée deutéroporphyrine,
Ra = -CH₂CH₂COORb avec Rb qui représente un atome d'hydrogène ou un groupe méthyle, éthyle, n-propyle ou i-propyle, alors nommées coproporphyrine III,
Ra = -C(O)CH₃, alors nommée diacétyldeutéroporphyrine.

Ces porphyrines peuvent être utilisées sous la forme de sels, par exemple d'un sel avec un métal alcalin au niveau des deux fonctions acide, tel qu'un sel de sodium.

Il est également possible, en fonction des applications, que ces porphyrines soient utilisées sous une forme complexée, par exemple avec un métal tel que Fe, ou encore un sel métallique tel que FeCl ou FeOH. Le complexe de la protoporphyrine IX avec Fe est nommé Hème, celui avec FeOH est nommé Hematine et celui avec FeCl Hémine.

Ces porphyrines sont le plus souvent préparées par hémisynthèse, ce qui pose le problème des impuretés d'origine animale, notamment, qui peuvent être présentes. Pour certaines applications, par exemple dans le cas de la protoporphyrine (IX), ou de son sel de sodium, qui peut être utilisé dans des milieux de culture cellulaire, il est souhaité de disposer d'un procédé de préparation totalement synthétique qui ne mette en oeuvre que des produits d'origine synthétique. Certains procédés de préparation par synthèse chimique de ces composés ont déjà été proposés. Les publications de JCS Perkin I, 1974, 1771-1781 et 1188-1194, par exemple, décrivent la préparation de la protoporphyrine IX. Une méthode connue pour préparer la protoporphyrine, à laquelle il est fait référence dans ces publications, est nommée la voie MacDonald et consiste à coupler, en présence d'un cation métallique M⁺ tel que Zn²⁺ ou Fe³⁺, les deux pyrrométhanes suivants (A) et (B) : 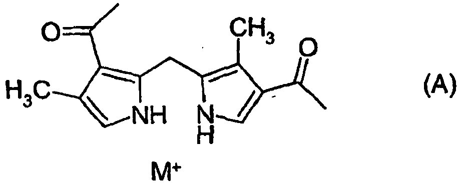 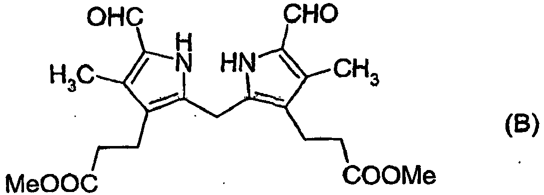 pour conduire à une structure porphodiméthane (C) : 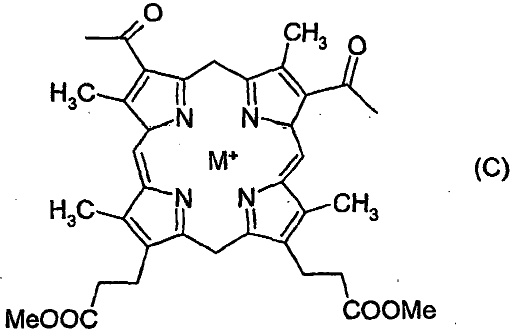 qui doit ensuite être oxydée pour former la porphyrine métallée (D) :

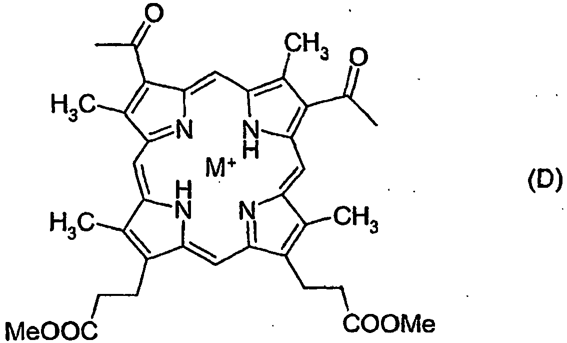

Une telle méthode est notamment décrite dans Science of Synthesis Houben-Weyl, vol 17, 1081-1235 et dans The porphyrin Handbook, vol.1, synthesis and Chemistry, Academic Press, Boston, 2000.

Il est ensuite nécessaire de démétaller la porphyrine, en présence d'acide sulfurique, si cette dernière doit être utilisée sous forme libre. Cette dernière étape notamment n'est pas quantitative et la porphyrine obtenue ne présente pas un degré de pureté satisfaisant. La fonction -C(O)CH₃ doit également être transformée en-CH=CH₂.

Dans ce contexte, la présente invention se propose de fournir un nouveau procédé de préparation synthétique exempt de tout contaminant d'origine animale, ne faisant appel qu'à des produits d'origine synthétique, qui soit adapté, en particulier, à la synthèse de la protoporphyrine IX, de la mesoporphyrine, de l'hématoporphyrine, de la deutéroporphyrine, des coproporphyrines III, de la diacétyldeutéroporphyrine, éventuellement sous forme de sels. Ce procédé se doit notamment de permettre leur production avec de hauts rendements et un degré de pureté élevé. Le procédé selon l'invention se doit également d'être facilement industrialisable et présenter une bonne rentabilité. Le procédé développé dans le cadre de l'invention permet, également, d'éviter la formation intermédiaire d'une porphyrine métallée.

Dans ce contexte, l'invention concerne un procédé de préparation d'une porphyrine de formule (I), éventuellement sous la forme d'un sel : 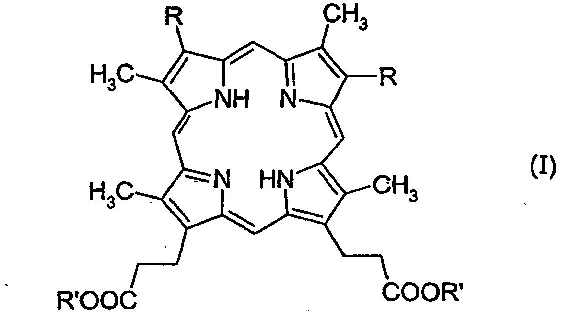 dans laquelle :
- R représente un atome d'hydrogène ou un groupe choisi parmi : -CH=CH₂, -CH₂-CH₃, -CH(OH)CH₃, -C(O)CH₃, -CH₂CH₂COOR'a avec R'a qui représente un atome d'hydrogène ou un groupe méthyle, éthyle, n-propyle ou i-propyle,
- R' représente un atome d'hydrogène ou un groupe R'b choisi parmi méthyle, éthyle, n-propyle ou i-propyle,
   comprenant :
- une étape de condensation, en milieu acide, entre un dipyrrométhane de formule (II) : 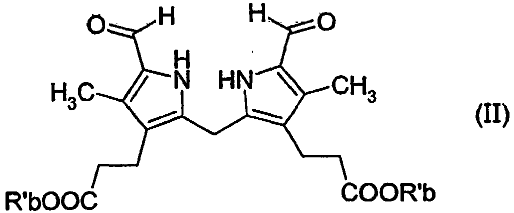 dans laquelle R'b est tel que défini précédemment pour (I), et un dipyrrométhane de formule (III) : 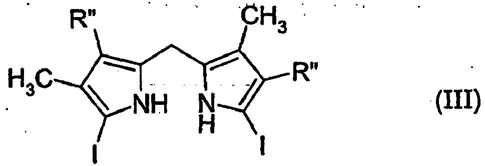 dans laquelle R" est identique à R tel que défini précédemment pour (I) ou est un groupe précurseur de R, pour former la porphyrine de formule (I') : 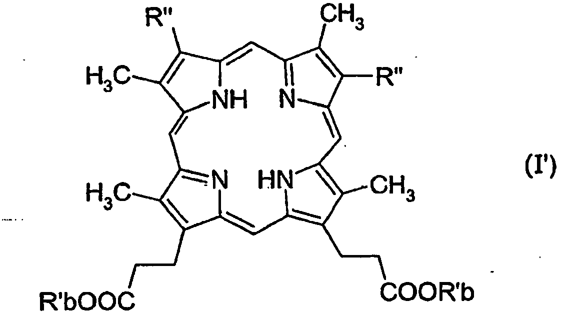 dans laquelle R" et R'b sont tels que définis précédemment pour (II) et (III), suivie :
   - lorsque R" est un groupe précurseur de R, de la transformation des groupes R" en R,
   - lorsque R'=H, d'une élimination du groupe R'b pour former des groupes -COOH, éventuellement sous forme de sel.

Le procédé selon l'invention permet d'obtenir des porphyrines présentant une solubilité satisfaisante, notamment en solution aqueuse.

A titre d'exemple de sels avec des porphyrines de formule (I), on peut par exemple citer les sels avec une base organique ou minérale. En particulier de tels sels peuvent être formés avec les porphyrines de formule (I) qui comprennent une fonction acide carboxylique, il s'agit de préférence d'un sel de métal alcalin, en particulier un sel de sodium, potassium ou lithium, ou d'un sel d'ammonium, d'un sel d'amine organique, ou d'un sel d'acide aminé telles que l'arginine ou la lysine.

Il est également possible de former des sels des porphyrines de formule (I) avec un acide minéral ou organique qui permettent, par exemple, une séparation ou une cristallisation convenable des composés de formule (1), ainsi que des sels pharmaceutiquement acceptables. En tant qu'acide approprié, on peut citer: l'avide picrique, l'acide oxalique ou un acide optiquement actif, par exemple un acide tartrique, un acide dibenzoyltartrique, un acide mandélique ou un acide camphosulfonique, et ceux qui forment des sels physiologiquement acceptables, tels que le chlorhydrate, le bromhydrate, le sulfate, l'hydrogénosulfate, le dihydrogénophosphate, le maléate, le fumarate, le 2-naphtalènesulfonate, le paratoluènesulfonate.

Les sels des composés de formule (I) sont préparés selon des techniques bien connues de l'homme de l'art, en incorporant l'étape correspondante de formation du sel souhaité, par action de la base ou de l'acide correspondant, de préférence en étape finale, au procédé selon l'invention.

Le procédé selon l'invention est illustré SCHEMA 1 ci-après dans lequel R, R" et R'b sont tels que définis pour les composés de formule (II), (III) et (I).

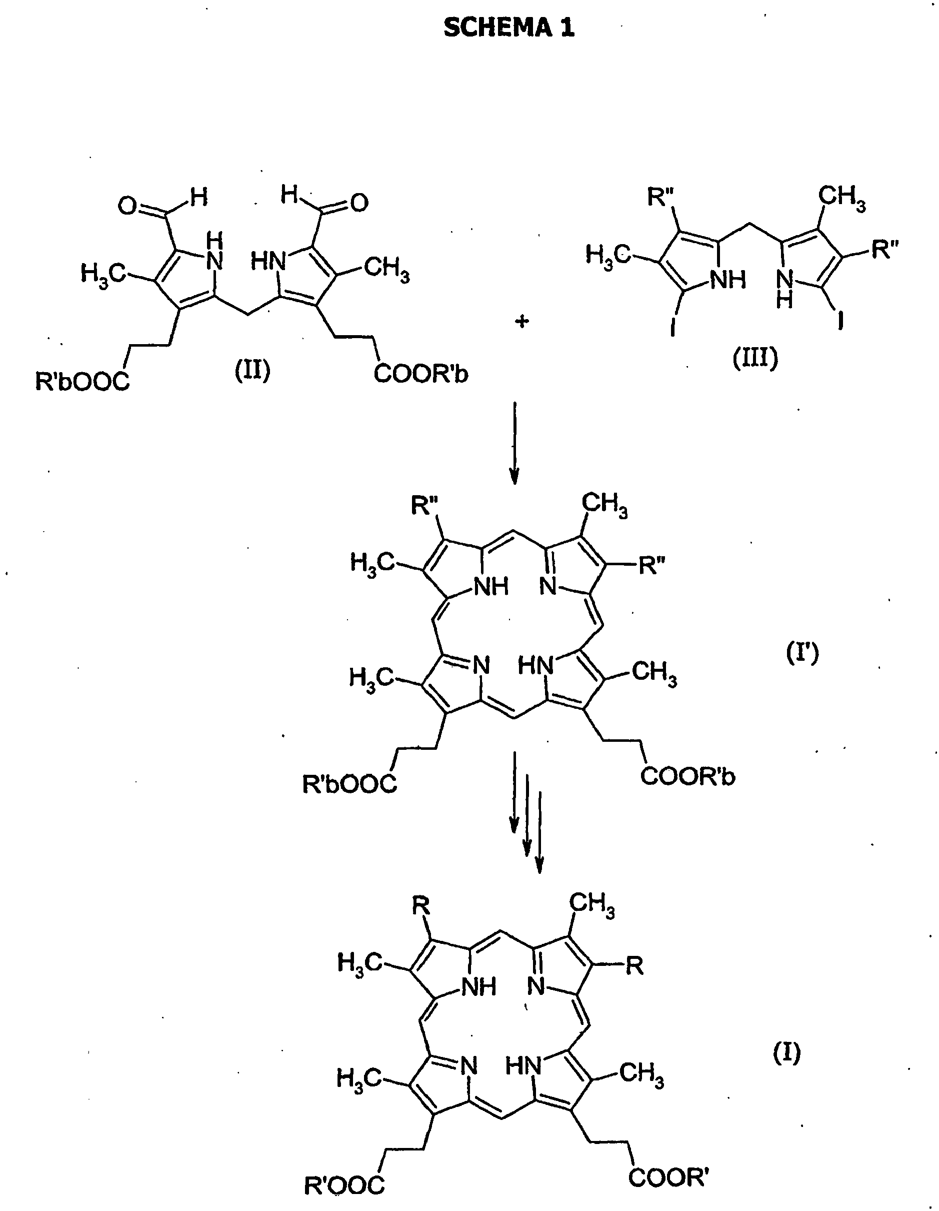

Généralement R" est un atome d'hydrogène ou un groupe chosi parmi : - CH=CH², - CH²-CH³, CH(OH)CH³, -C(O)CH³, -CH²CH²OH, -CH²CH²Cl, -CH₂CH₂OC(O)CH₃ -CH₂CH₂COOR'a avec R'a qui représente un atome d'hydrogène ou un groupe méthyle, éthyle, n-propyle ou i-propyle

En fonction de la nature du groupement R, le couplage peut être réalisé entre un composé (II) et un composé (III) dans lequel R"=R ; c'est par exemple le cas quand R = H, -CH₂-CH₃, -CH(OH)CH₃, -C(O)CH₃, -CH₂CH₂COOR'a avec R'a qui représente un atome d'hydrogène ou un groupe méthyle, éthyle, n-propyle ou i-propyle.

Si le groupe R'b=R', le composé (I') directement obtenu après l'étape de condensation est le composé (I) souhaité, sans qu'aucune étape supplémentaire ne soit nécessaire. Si le composé R'b est différent de R', ce qui est le cas quand R' = H ou bien dans les cas où les fonctions acide sont sous la forme d'un sel, par exemple, avec un métal alcalin tel que Na⁺ ou K⁺, le couplage est suivi de la déprotection de la fonction acide par élimination du groupe R'b, afin de transformer le composé (I'), en composé (I).

Le couplage peut également être effectué avec un composé (III) dans lequel R" est un groupe précurseur de R. Par groupe précurseur de R, on entend un groupe qui, après une ou plusieurs réactions chimiques, conduit au groupe R désiré. A titre d'exemple de tels groupes précurseurs, notamment pour le groupe -CH₂=CH₂, on peut, par exemple, citer les groupes -C(O)CH₃, -CH(OH)CH₃, -CH₂CH₂OH, - CH₂CH₂OC(O)CH₃ ou -CH₂CH₂Cl, les groupes -C(O)CH₃ et -CH(OH)CH₃, étant particulièrement préférés. Le couplage avec le composé (III) conduit à un composé (I') : 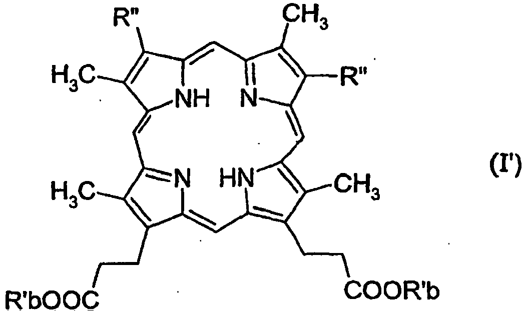 dans lequel R" représente un groupe précurseur de R. Le groupe précurseur de R doit alors être transformé pour conduire au groupe R souhaité, en une ou plusieurs étapes. C'est, par exemple, le cas pour la préparation des composés de formule (I) dans lesquels R = -CH=CH₂, -CH₂-CH₃, -CH(OH)CH₃, ou H. Dans le cas de tels groupements, une des méthodes consiste à effectuer le couplage avec un composé (III) dans lequel R"=-C(O)CH₃ qui est ensuite transformé, après l'étape de condensation entre les composés (II) et (III), pour obtenir le groupement R souhaité.

Par ailleurs, en fonction de la nature du groupement R', le couplage peut être réalisé entre un composé (III) et un composé (II) dans lequel R'=R'b. Par contre dans le cas où R' = H, ou bien dans les cas où les fonctions acide sont sous la forme d'un sel, par exemple, avec un métal alcalin tel que Nₐ⁺ ou K⁺, le couplage est suivi de la déprotection de la fonction acide par élimination du groupe R'b.

Lorsqu'après l'étape de couplage entre les composés (II) et (III), les deux étapes, à savoir la transformation du groupe R" eh R et l'élimination du groupe R'b, sont nécessaires, la déprotection de la fonction acide peut intervenir, avant ou après le transformation du groupe R" en R. Il est, néanmoins, préféré d'éliminer le groupe R'_{b}, après la transformation du groupe R" en R, les fonctions esters améliorant la solubilité dans les solvants de réaction.

A la différence de la méthode de MacDonald de l'art antérieur, l'étape de condensation entre les composés (II) et (III) est réalisée en l'absence de métal, sel ou dérivé métallique, susceptible de se complexer avec la porphyrine (I') formée.

Dans le cadre de l'invention, pour la préparation d'un composé de formule (I) dans laquelle R représente un atome d'hydrogène ou un groupe choisi parmi : -CH₂- CH₃, -CH(OH)CH₃, -C(O)CH₃, -CH₂CH₂COOR'a avec R'a qui représente un atome d'hydrogène ou un groupe méthyle, éthyle, n-propyle ou i-propyle, on pourra opérer le couplage, soit avec un composé (III) dans lequel R" représente le groupement R final, soit avec un composé (III) dans lequel R" représente un groupe précurseur du groupement R final.

Pour la préparation d'un composé de formule (I) dans laquelle R représente un groupe -CH=CH₂, on préférera utiliser :
une étape de condensation, en milieu acide, entre un dipyrrométhane de formule (II) : 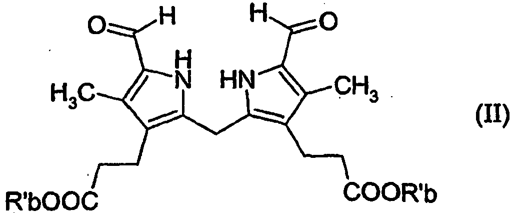 dans laquelle R'b est tel que défini précédemment pour (I), et un dipyrrométhane de formule (III) : 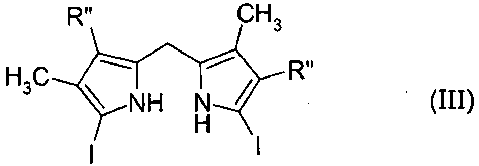 dans laquelle R" est un groupe précurseur de R, par exemple un groupe -CH(OH)CH₃ ou - C(O)CH₃,
   suivie de la transformation des groupes R" en R,
   et lorsque R'=H, de l'élimination des groupes R'b pour obtenir les groupes -COOH, éventuellement sous forme de sel.

Le procédé selon l'invention est particulièrement adapté à la synthèse de la protoporphyrine et de ses sels, en particulier son sel de sodium de formule (IC.2, 2Na) :

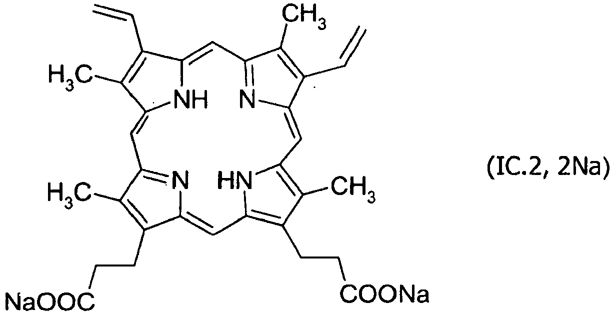

Dans le cas de la préparation d'un composé de formule (IC) : 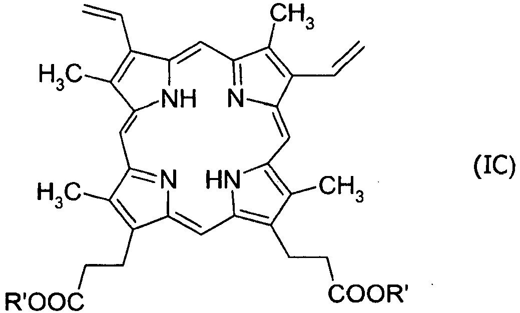 dans laquelle R' est tel que défini pour (I), ou un de ses sels, par exemple, avec un métal alcalin, on réalise, de façon avantageuse, un couplage entre un pyrrométhane de formule (II) : 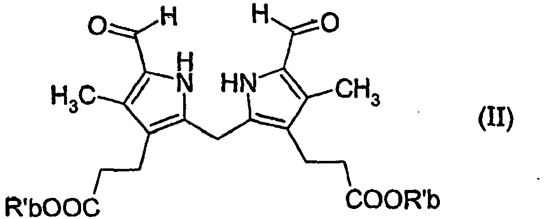 dans laquelle R'b est tel que défini précédemment pour (I), et représente de préférence un groupe méthyle,
et un dipyrrométhane de formule (IIIa) : 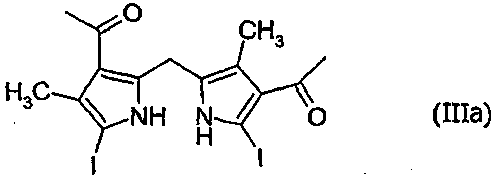 pour former le composé de formule (Ia) : 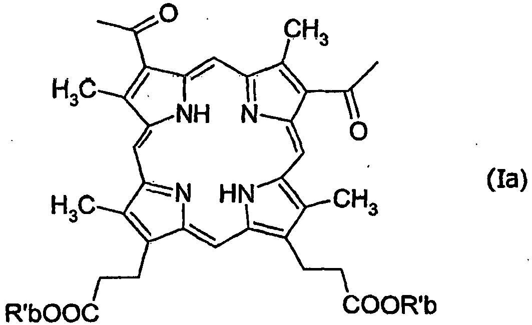 dans laquelle R'b est tel que défini pour (I), et représente, de préférence, un groupe méthyle,
suivi :
- d'une réduction de la fonction -C(O)CH₃, conduisant à la formation de la porphyrine de formule (Ib) : 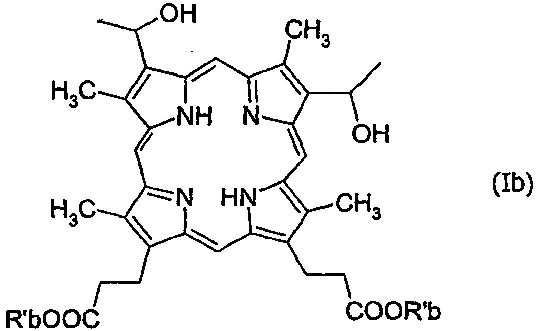 dans laquelle R'b est tel que défini pour (I), et représente, de préférence, un groupe méthyle,
- suivi d'une réaction d'élimination transformant les groupes -CH(OH)CH₃ en -CH=CH₂,
- et, dans le cas où R' représente un atome d'hydrogène, d'une étape de déprotection de la fonction -COOH par hydrolyse,
ou, dans le cas où le composé (IC) que l'on souhaite former est sous la forme d'un sel avec un métal alcalin, d'une étape de saponification.

Il est à noter que, dans le cadre de l'invention, le composé de formule (Ib) comprend deux carbones asymétriques et peut se trouver sous la forme d'un mélange d'isomères ou d'un isomère pur.

Ce procédé de préparation des composés de formule (IC) est illustré **SCHEMA 2** ci-dessous dans lequel R'b est tel que défini pour les composés de formule (II):

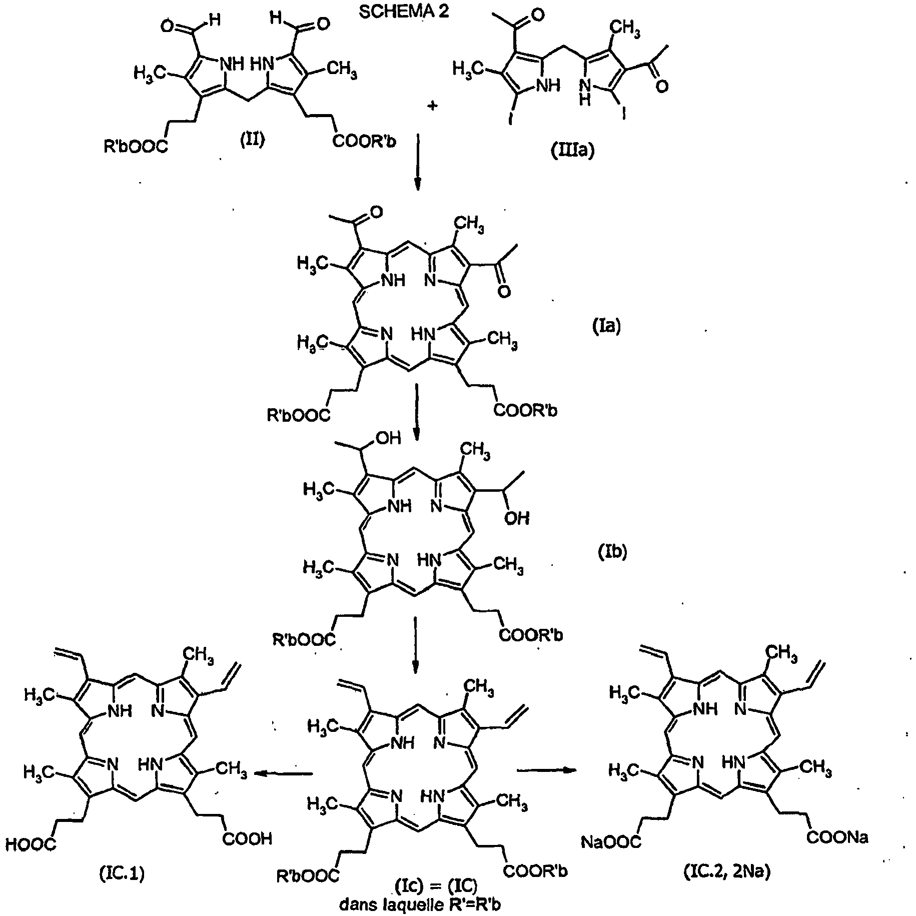

Dans le cas de la préparation de la protoporphyrine IX sous la forme du sel de sodium de formule (IC.2,2Na) : 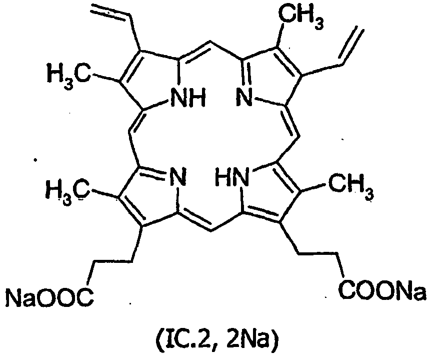 la dernière étape du procédé consiste en la saponification des deux groupes -COOR'b du composé de formule : 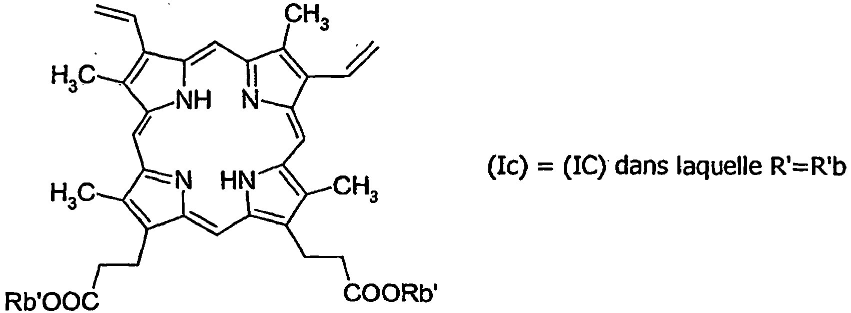 dans laquelle R'b est tel que défini pour (I), et représente, de préférence, un groupe méthyle. Cette étape de saponification peut être réalisée par action de soude, en présence de méthanol. Par exemple, l'étape de saponification est réalisée dans le dichloromethane à reflux. Selon une variante non préférée, il pourrait également être prévu que cette étape de saponification soit réalisée, après le couplage entre les composés (II) et (IIIa), mais avant l'obtention du groupe -CH=CH₂ souhaité.

La réaction de condensation entre les deux pyrrométhanes (II) et (III) ou (IIIa) est réalisée, de préférence, en présence d'un acide choisi parmi les acides carboxyliques, l'acide trifluoroacétique, l'acide chlorhydrique, l'acide trichlorométhanesulfonique, l'acide méthanesulfonique, l'acide trifluorométhanesulfonique, l'acide tetrafluoroborique, l'acide bromhydrique, l'acide iodhydrique. L'acide est, préférentiellement, un acide fort, de préférence l'acide trifluoroacétique ou l'acide trichlorométhanesulfonique. L'acide est, de préférence, utilisé en excès par rapport au pyrrométhane (II), par exemple 2 équivalents molaires d'acide par équivalent molaire de pyrrométhane (II).

De façon avantageuse, la réaction de condensation entre les deux pyrrométhanes (II) et (III) ou (IIIa) est réalisée en présence d'un agent dessiccateur, destinée à capter les molécules d'eau. A titre d'agent dessiccateur, on peut citer les anhydrides, les tamis moléculaires, l'acide sulfurique, l'anhydride acétique étant préféré. Dans le cas de l'utilisation d'anhydride acétique, celui-ci est, de préférence, présent en excès, par exemple, d'au moins 10, préférentiellement d'au moins 50 équivalents molaires par rapport au pyrrométhane (II) (c.a.d. par équivalent molaire de pyrromethane II).

Il sera également avantageux de réaliser la condensation avec sensiblement un équivalent ou un léger excès de pyrrométhane (II), par rapport au pyrrométhane (III) ou (IIIa). Généralement, la réaction de condensation entre les deux pyrrométahnes est réalisée selon un ratio en équivalent molaire compris entre 1 et 1,2. La réaction de condensation entre les deux pyrrométhanes (II) et (III) ou (II) et (IIIa) est, par exemple, effectuée à une température de 10 à 50°C, de préférence de 20 à 25 °C, dans un solvant protique, tel que l'acide acétique.

Les réactions suivants le couplage, permettant d'obtenir le groupe R souhaité, font appel à des méthodes connues.

La réaction d'élimination transformant les groupes -CH(OH)CH₃ en -CH=CH₂ est réalisée, avantageusement, en présence d'un halogénure d'acide, de préférence un chlorure d'acide tel que le chlorure de benzoyl. Par exemple, la réaction d'élimination transformant les groupes -CH(OH)CH₃ en -CH=CH₂, est réalisée dans un solvant polaire aprotique tel que le DMSO (diméthyl sulfoxyde), l'acétone ou de préférence le DMF (diméthyl formamide), de préférence à une température de 50 à 200°C pendant une durée de 30 minutes à 3 heures, et préférentiellement à une température de 80 à 120°C pendant une durée de l'ordre d'une heure.

La réduction de la fonction -C(O)CH₃ en -CH(OH)CH₃ est, avantageusement, réalisée en présence d'un hydrure, de préférence, un borohydrure tel que NaBH₄ ou BH₃. De préférence, la réduction de la fonction -C(O)CH₃ est réalisée dans le dichlorométhane en présence de méthanol, par exemple à une température comprise entre 0 et 60°C, préférentiellement entre 20 et 30°C.

Par la suite, le composé de formule (I) dans lequel R= -CH=CH₂ peut être soumis à d'autres réactions chimiques, afin d'obtenir d'autres groupes R. Le composé de formule (I) dans lequel R= -CH₂-CH₃, par exemple, peut être obtenu à partir du composé de formule (I) correspondant dans lequel R= -CH=CH₂ par hydrogénation catalytique. Par exemple, on pourra utiliser la technique décrite dans Tetrahedron Letters 2006, 47(29), 5119-22, qui met en oeuvre un catalyseur RuCl₃ dans AcNMe₂ à une température de l'ordre de 80°.

De même, le composé de formule (I) dans lequel R=H peut être obtenu à partir du composé de formule (I) correspondant dans lequel R= -C(O)CH₃ en formant intermédiairement le composé de formule (I) dans lequel R= -CH(OH)CH₃ qui est ensuite desacétylé par action de BF₃ en présence de HS-(CH₂)₂-SH, par exemple en utilisant la méthode décrite dans JOC, 1983, 48(24), 4779-81 ou J.Chem Soc, Chemical Communications, 1981, (6), 253-4.

Selon une de ses variantes, le procédé selon l'invention peut donc être mis en oeuvre pour la préparation des composés de formule (IA) : 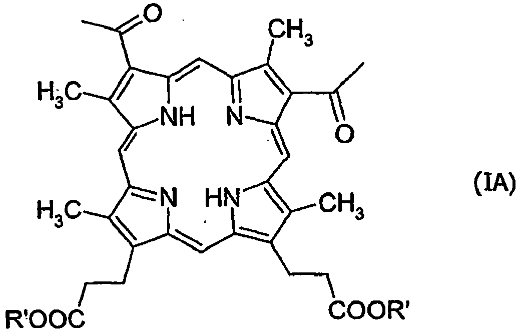 dans laquelle R' est tel que défini pour (I), ou un de ses sels, par exemple, avec un métal alcalin,
par couplage du pyrrométhane de formule (II) : 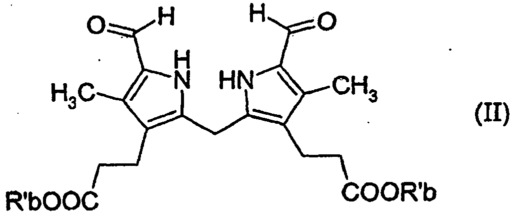 dans laquelle R'b est tel que défini précédemment pour (I), et représente de préférence un groupe méthyle,
avec un dipyrrométhane de formule (IIIa) : 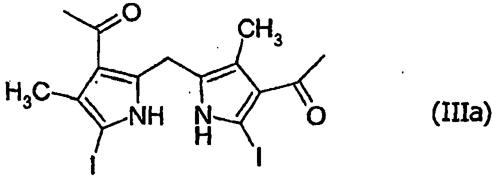 suivi, dans le cas où R' représente un atome d'hydrogène, d'une étape de déprotection de la fonction -COOH par hydrolyse,
et/ou, éventuellement dans le cas où l'on souhaite former le composé (Ia) sous la forme d'un sel avec un métal alcalin, d'une étape de saponification.

Le procédé selon l'invention peut également être mis en oeuvre pour la préparation du composé de formule (IB) : 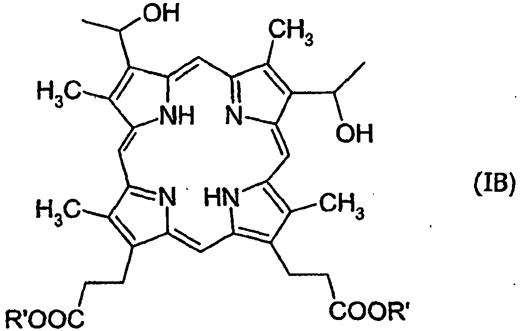 dans laquelle R' est tel que défini pour (I), ou un de ses sels, par exemple, avec un métal alcalin,
par couplage du pyrrométhane de formule (II) : 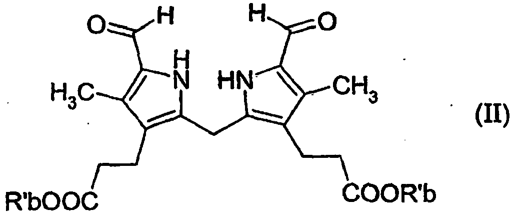 dans laquelle R'b est tel que défini précédemment pour (I), et représente, de préférence, un groupe méthyle,
avec un dipyrrométhane de formule (IIIa) : 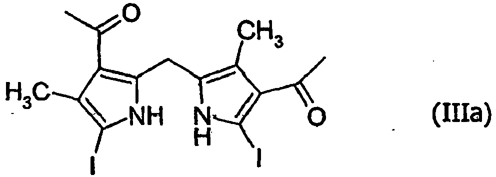 pour former un composé de formule (Ia) : 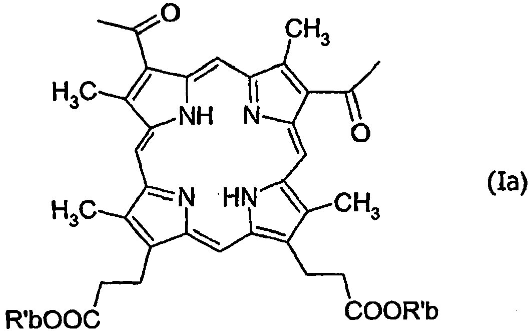 dans laquelle R'b est tel que défini précédemment pour (I), et représente, de préférence, un groupe méthyle,
suivi, d'une réduction de la fonction -C(O)CH₃, pour former la fonction -CH(OH)CH₃ et, dans le cas où R' représente un atome d'hydrogène, d'une étape de déprotection de la fonction -COOH, par hydrolyse,
et/ou, dans le cas où l'on souhaite former un composé (IB) sous la forme d'un sel avec un métal alcalin, d'une étape de saponification.

Le composé (II) dans lequel R'b=Me est un composé connu, tout comme les composés (II) dans lesquels R'b est un groupe éthyle ou propyle (JCS Perkin I, 1974, 1188-1194 et 1771-1781). On pourra se référer pour leur synthèse, par exemple, à Austral. J. Chem. 1969, 22, 229, JCS, Chem. Comm. 1985, (8), 470-1, Org. Bioorg. Chem. (1972-1999), 1987, (2), 265-76, et J. Porphyrins and Phtalocyamines, 2002, 6 (9+10), 607-16.

Par contre, les pyrrométhanes de formule (III) : 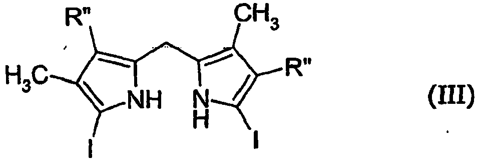 dans laquelle R" représente un groupe R choisi parmi un atome d'hydrogène ou un groupe choisi parmi : -CH=CH₂, -CH₂-CH₃, -CH(OH)CH₃, -C(O)CH₃, -CH₂CH₂COOR'a avec R'a qui représente un atome d'hydrogène ou un groupe méthyle, éthyle, n-propyle ou i-propyle, ou bien un groupe précurseur de R, sont des composés nouveaux et font partie intégrante de l'invention.
Parmi les composés de formule (III), on peut citer ceux dans lesquels R" représente un groupe précurseur de -CH=CH₂ choisi parmi : -C(O)CH₃, -CH(OH)CH₃, -CH₂CH₂OH, - CH₂CH₂OC(O)CH₃ ou -CH₂CH₂Cl.

La transformation du groupe -CH₂CH₂OH, -CH₂CH₂OC(O)CH₃ ou -CH₂CH₂Cl en -CH=CH₂ se fait selon les méthodes classiques d'élimination bien connues de l'homme du métier. Par exemple, le groupe -CH₂CH₂Cl peut être traité par action de potasse alcoolique comme décrit dans J.C.S. Perkin I, 1974, 1771-1781.

A titre d'exemple de tels composés, on peut citer le pyrrométhane de formule (IIIa) :

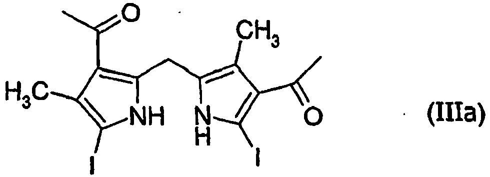

Les composés (III) peuvent être préparés selon le SCHEMA 3 ci-après :

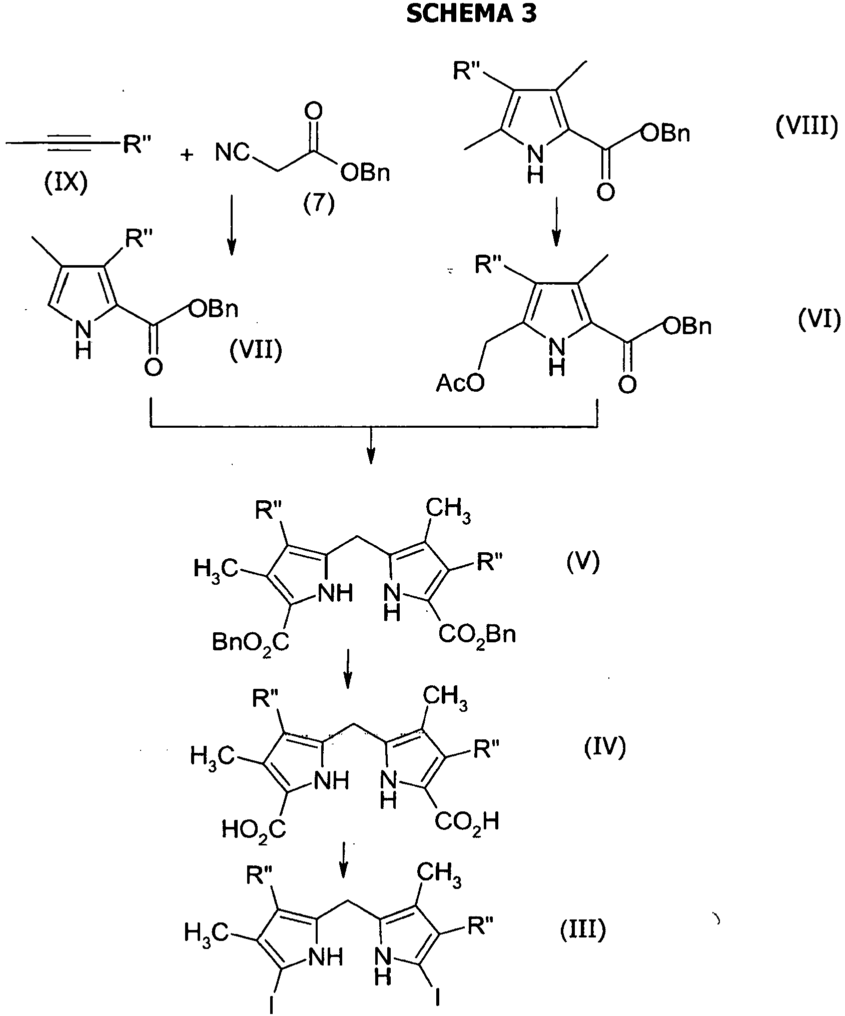

Dans le cas où R" représente un groupe -C(O)CH₃, le composé (IX) est, par exemple, préparé par réaction du triméthylsillylpropyne et de chlorure d'acétyle, en présence de trichlorure d'alluminium.

Le couplage entre les composés (VII) et (VI) est, de préférence, réalisé en milieu acide, par exemple en présence de TFA, HCl, MeSO₃H, SnCl₄, HBF₄ et, avantageusement, en présence de HBF₄ ou de CF₃SO₃H. Par exemple, le couplage est réalisé dans un solvant tel que l'acide acétique, ou de préférence le dichloroéthane, par exemple à une température comprise entre 50 et 150°C, notamment de l'ordre de 90 à 100°C, pendant 1 à 12 heures.

L'obtention du composé (IV) par débenzylation du composé (V) est par exemple réalisée par hydrogénation catalytique. A titre illustratif, un catalyseur métallique, tel qu'un catalyseur à base de nickel, platine ou de préférence palladium, peut être utilisé.

Les composés de formule (VIIa), (Va), (IVa), (composés VII, V, IV respectivement, dans lesquels R" = -C(O)CH₃) suivants : 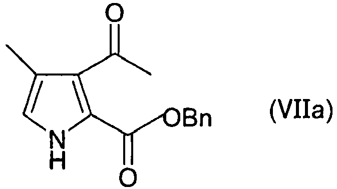 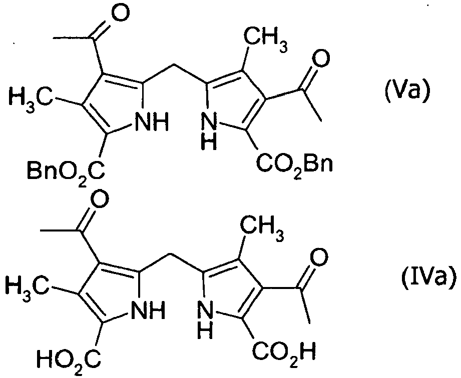 sont également des intermédiaires nouveaux qui font partie intégrante de l'invention.

Pour la préparation du composé (IV) dans lequel R"= -CH₂CH₂COOR'a avec R'a tel que défini pour (I), on pourra se référer à JCS, Perkin Trans 1 Org and Bioorg Chem. 1987 (2), 299-305. Le composé (IV) dans lequel R"= -CH₂CH₃ est, quant à lui, décrit dans Zhurnal Obshcheikhimii 1966, 36(7), 1208-10.

La présente invention concerne également un procédé de préparation d'une porphyrine de formule (I), éventuellement sous la forme d'un sel : 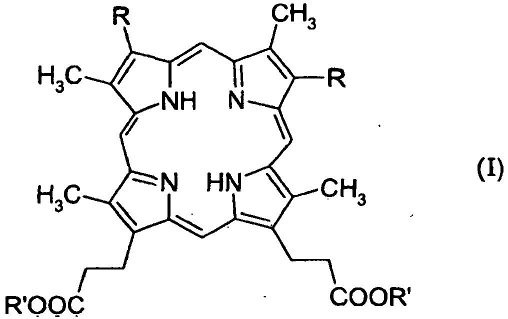 dans laquelle :
- R représente un atome d'hydrogène ou un groupe choisi parmi : -CH=CH₂, -CH₂-CH₃, - CH(OH)CH₃, -C(O)CH₃, -CH₂CH₂COOR'a avec R'a qui représente un atome d'hydrogène ou un groupe méthyle, éthyle, n-propyle ou i-propyle,
- R' représente un atome d'hydrogène ou un groupe R'b choisi parmi méthyle, éthyle, n-propyle ou i-propyle,
sous la forme d'un complexe métallique, par exemple du fer, galium, nickel, zinc, palladium, cobalt, calcium ou magnésium, dans lequel une étape de complexation d'une porphyrine, formée après l'étape de condensation entre les composés (II) et (III) ou (IIIa), est mise en oeuvre, par action du métal sélectionné ou d'un sel ou dérivé métallique du métal sélectionné.

De tels complexes répondent, notamment, à la formule (I") suivante : 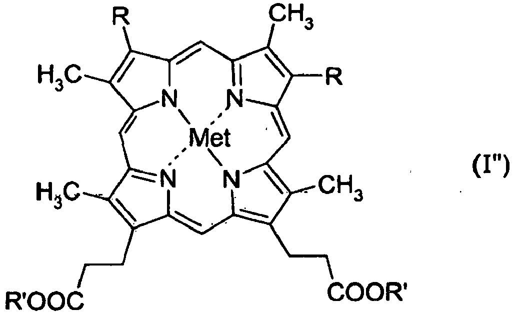 dans laquelle :
- R représente un atome d'hydrogène ou un groupe choisi parmi : -CH=CH₂, -CH₂-CH₃, - CH(OH)CH₃, -C(O)CH₃, -CH₂CH₂COOR'a avec R'a qui représente un atome d'hydrogène ou un groupe méthyle, éthyle, n-propyle ou i-propyle,
- R' représente un atome d'hydrogène ou un groupe R'b choisi parmi méthyle, éthyle, n-propyle ou i-propyle, et
- Met représente un métal bivalent M(II) ou un sel métallique d'un métal trivalent M(III)X, avec X qui représente Cl ou OH et M qui représente le fer, le galium, le nickel, le zinc, le palladium, le cobalt, le calcium ou le magnésium.

La porphyrine de formule (I) ou le porphyrine de formule (I') obtenue, dans le procédé selon l'invention peut être mise à réagir avec un métal, un sel de métal ou un hydroxyde de métal, du type chlorure, hydroxyde, acétate, sulfate notamment, le métal étant, par exemple, choisi parmi le fer, le galium, le nickel, le zinc, le palladium, le cobalt, le calcium ou le magnésium, de manière à obtenir la porphyrine de formule (I) sous la forme d'un complexe métallique. Cette métallation intervient, nécessairement après le couplage des pyrrométhanes (II) et (III) ou (IIIa).

L'étape de complexation est, de préférence, réalisée, en étape finale, sur la porphyrine de formule (I), éventuellement sous la forme d'un sel. Pour la formation de tels complexes, on pourra se référer aux publications suivantes, décrivant la formation de complexes métalliques dans le cas où R" représente un groupe -CH=CH₂, ou aux méthodes connues de complexation pour l'obtention de l'hème, l'hémine ou l'hématine, ces méthodes étant adaptées aux différents groupes R et R' tels que définis pour l'ensemble des composés de formule I :
- Journal of Photochemistry and Photobiology, A : Chemistry, 172(1), 55-61, 2005, qui décrit la formation du complexe suivant : 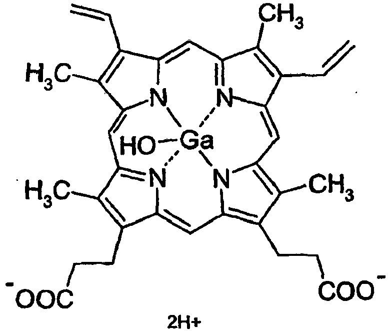 par action de GaCl₃, suivi d'une hydrolyse avec de la potasse dans le méthanol, cette méthode pouvant être adaptée de façon analogue à la formation de l'Hématine, par action de FeCl₃,
- Journal of Molecular Catalysis A: Chemical, 235(1-2), 185-193, 2005, qui décrit la formation du complexe suivant : 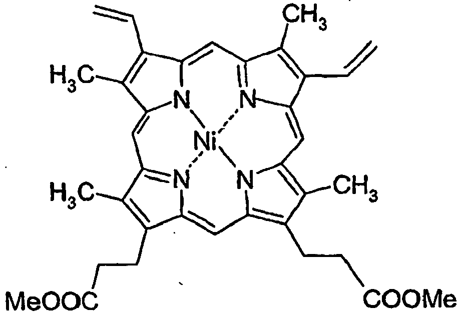 par action de Ni(OAc)₂, dans le DMF.
- Journal of Molecular Catalysis A: Chemical, 235(1-2), 185-193, 2005, qui décrit la formation du complexe C : 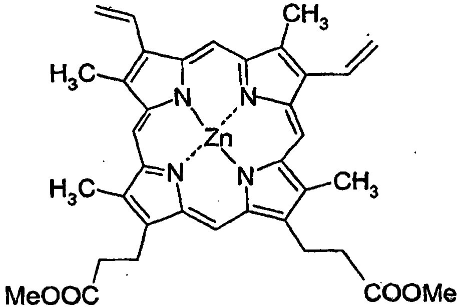 par action de Zn(OAc)₂, dans un mélange méthanol/trichlorométhane.
- Faming Zhuanli Shenqing Gongkai Shuomingshu, 1418885, qui décrit la formation du complexe suivant : 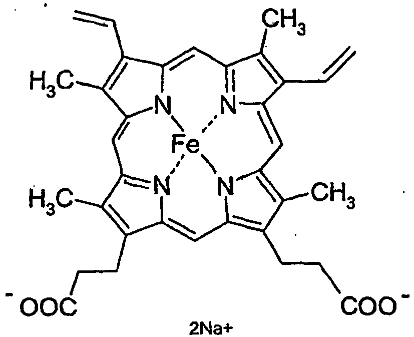 par traitement à l'acide sulfurique, puis d'un traitement avec FeSO₄, suivi d'un, traitement à la soude.
- Tetrahedron Letters, 27(30), 3521-4, 1986, qui décrit la formation du complexe suivant : 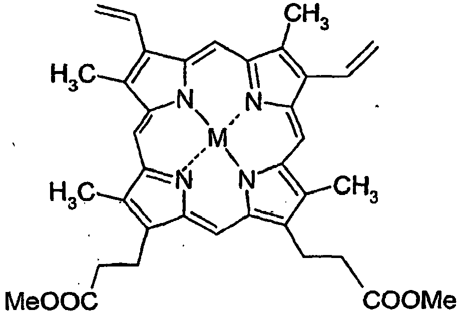 par action de BF₄⁻, Ph₂S⁺(CH₂Ph) dans le dichlorométhane, suivi de l'action de PdCl₂ dans le cas où M=Pd, de NiCl₂ dans le cas où M = Ni, ou de CoCl₂ dans le cas où M = Co dans le méthanol.
- Journal of Organic Chemistry, 51(24), 4660-7, 1986, et Journal of Organic Chemistry, 51 (5), 666-71, 1986 qui décrivent la formation du complexe suivant : 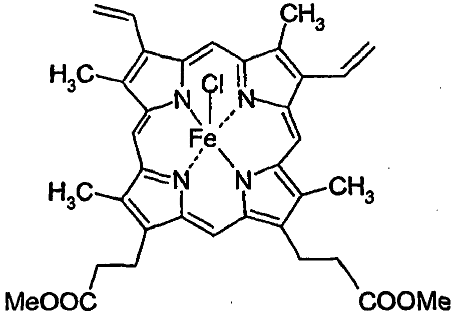
par action de FeCl₂, dans un mélange dichlorométhane/acétonitrile sous atmosphère inerte.

Les exemples et préparations ci-après permettent d'illustrer l'invention.

Le **SCHEMA 4** ci-après résume les différents composés préparés et les étapes du procédé mis en oeuvre dans les préparations et exemples.

Les abréviations suivantes sont utilisées.
Bn = benzyle, Et = éthyle, Ac = -C(O)Me, Me = méthyle
Bn =

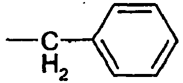

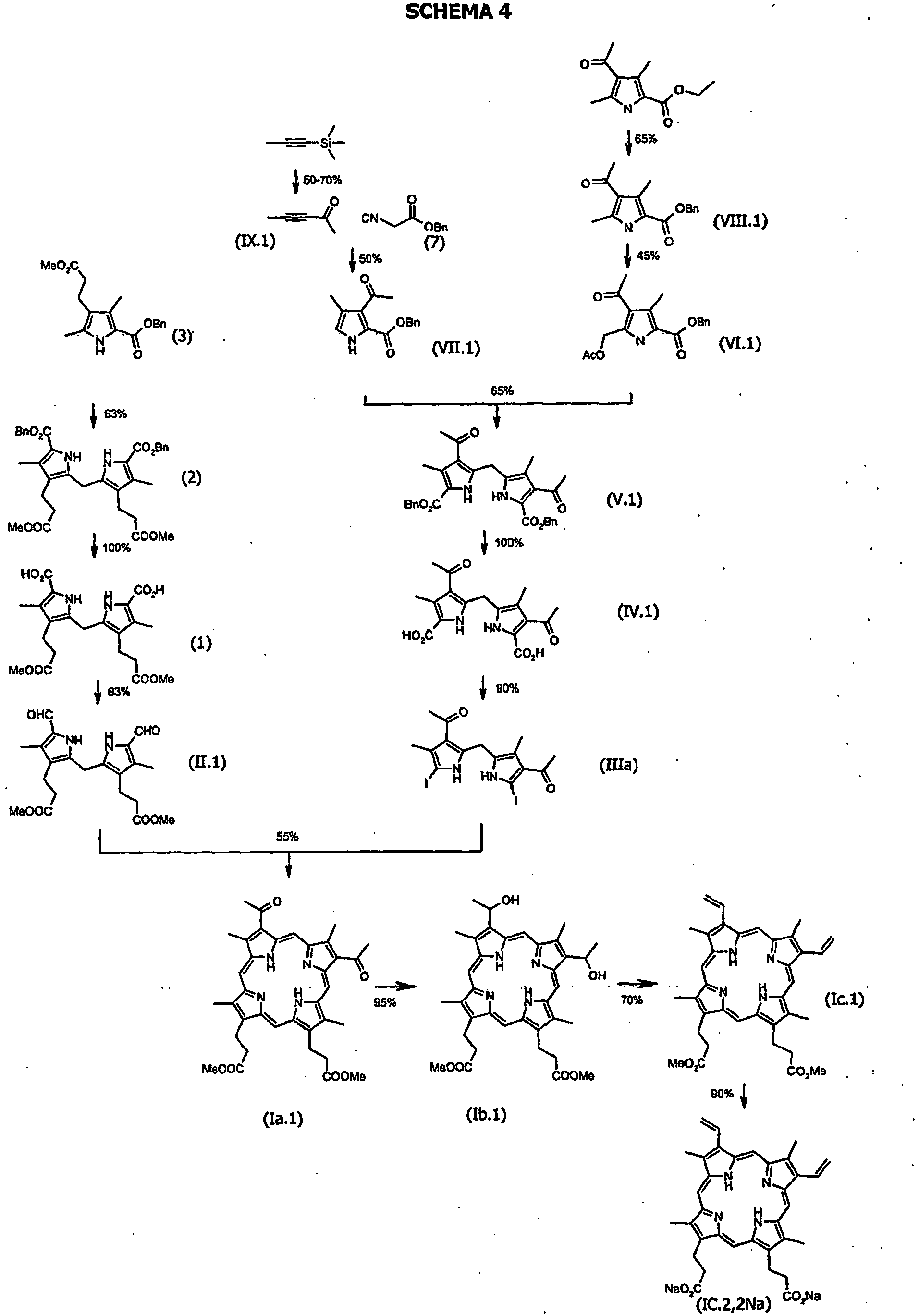

### PREPARATION 1

### Synthèse du pyrrole de formule (3) :

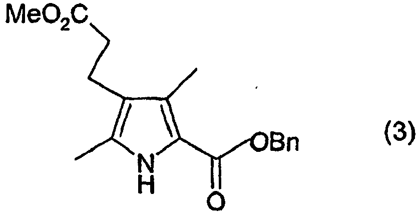

### a) Préparation du composé de formule (6) :

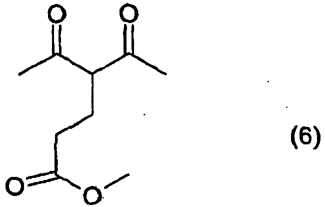

Un ballon Keller de 4,5 L équipé d'un réfrigérant à reflux, d'un thermomètre, d'une ampoule à brome et d'un conduit à argon est chargé avec de l'éthanol (2 L). A la température ambiante, du sodium (2,2 g) est dissous progressivement pour donner une solution limpide qui est refroidie à 0°C. De l'acétonate d'acétyl (500,0 g, 5,0 mol) est ajouté goutte à goutte en 10 min, ce qui conduit à un dégagement gazeux. 430g d'acrylate de méthyle sont ajoutés goutte à goutte à la solution jaune clair à 0°C en 10 min, ce qui conduit à un dégagement gazeux. Le mélange réactionnel est mis à chauffer à la température ambiante puis est chauffé à reflux pendant 1 h. La conversion est suivie par HPLC. Le mélange est mis à refroidir à la température ambiante. De l'acide acétique (3 mL) est ajouté et l'éthanol est éliminé par distillation sous pression réduite. La distillation du mélange brut (95-105°C, 2,5 mbar) donne une solution jaune clair (747,8 g, 80 %). L'analyse par RMN ¹H montre que le produit brut est un mélange ~1/1 de composé **(6)** et de 5-oxohexanoate de méthyle.

### b) Préparation du composé de formule (5) :

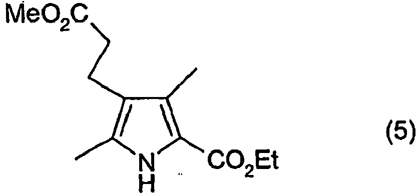

Un ballon Keller de 4,5 L équipé d'un réfrigérant à reflux, d'un thermomètre, d'une ampoule à brome et d'un conduit à argon est chargé avec de l'acide acétique (1,30 L) et chauffé à reflux. Une solution d'un mélange du composé **(6)** (248,71 g, 1,34 mol) et de chlorhydrate d'aminomalonate de diméthyle (367,50 g, 1,74 mol) dans l'acide acétique (0,85 L) est ajoutée goutte à goutte au mélange à reflux en 1 h. Le mélange est chauffé encore à reflux pendant 2,5 h. La conversion est suivie par HPLC. Le mélange réactionnel est mis à refroidir à la température ambiante et l'acide acétique est éliminé par distillation sous pression réduite. Le produit sombre brut est trituré avec de l'eau (4,5 L) qui est ajouté lentement par portions. Le mélange est agité mécaniquement pendant 1 h encore puis filtré et le gâteau de filtration est lavé avec de l'eau (1 L). La recristallisation du produit brut gris foncé est réalisée dans l'éthanol/eau (350/350 mL) par chauffage à reflux puis refroidissement à 10°C. Le précipité est isolé par filtration et lavé avec de l'éthanol/eau (4 x 100/100 mL). Le produit est séché sous pression réduite à la température ambiante pour donner le composé **(5)** (95,00 g, 28 %) sous forme d'un solide coloré pourpre clair.

RMN ¹H (300MHz, CDCl₃) : 1,34 (t,CH3), 2,21 (s,CH3), 2,27 (s,CH3), 2,43 (t,CH2), 2,70 (t,CH2), 3,66 (s,CH3), 4,29 (q,CH2), 8.60 (singulet large, NH2):

### c) Préparation du composé de formule (4) :

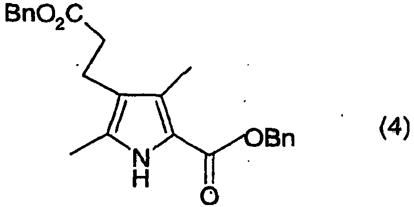

Un ballon Keller de 2,5 L équipé d'un réfrigérant à reflux/tête de distillation, d'un thermomètre, d'une ampoule à brome et d'un conduit à argon est chargé avec une solution de composé **(5)** (95,00 g, 0,38 mol) dans l'alcool benzylique (685 mL) et chauffé à 120°C, ce qui conduit au retrait azéotropique de quantités mineures d'eau. Le mélange est ensuite chauffé à 190°C. L'ampoule à brome est chargée avec une solution préparée séparément de sodium (3 g) dans l'alcool benzylique (70 mL). Cette solution est ajoutée par portions de 5 mL ce qui conduit à un reflux vigoureux du mélange réactionnel. Le méthanol et l'éthanol résultant sont retirés par distillation de manière semi-continue. La conversion est suivie par HPLC. Le mélange réactionnel est mis à refroidir à 150°C puis transféré sur un mélange de méthanol (0,85 L), d'eau (0,54 L) et d'acide acétique (10 mL). A 30°C, la cristallisation se produit rapidement. Le mélange est agité encore à la température ambiante pendant 1 h. Le produit est isolé par filtration. Le produit est séché sous pression réduite pour donner le composé **(4)** (113,30 g, 77 %) sous forme d'un solide blanc cassé.

RMN ¹H (300MHz, CDCl₃) : 2,16 (s,CH3), 2,27 (s,CH3), 2,47 (t,CH2), 2,71 (t,CH2), 4,70 (s,CH3), 5,08 (s,CH2), 5,28 (s,CH2), 7,40 (m,10H), 8,60 (singulet large,NH2).

### d) Préparation du composé de formule (3) :

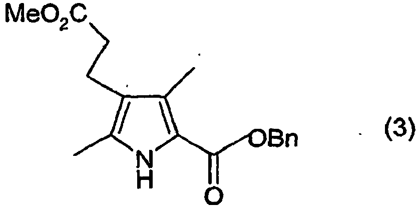

Un ballon Keller de 2,5 L équipé d'un réfrigérant à reflux, d'un thermomètre, d'une ampoule à brome et d'un conduit à argon est chargé avec une solution de sodium (2,8 g) dans le méthanol (710 mL). A la température ambiante, une solution de composé **(4)** (111,00 g, 0,28 mol) dans le THF (430 mL) est ajoutée goutte à goutte en 10 min. Le mélange est agité encore pendant 1 h. La conversion est suivie par HPLC. Après addition d'acide acétique (7 mL), les produits volatils sont retirés sous pression réduite. Le produit visqueux brut est dissous dans l'éthanol (490 mL) et de l'eau (280 mL) est ajoutée. Le mélange résultant est agité pendant 1 h à 0°C et le produit précipité est isolé par filtration. Le produit est lavé avec de l'éthanol/eau (250/250 mL) et séché sous pression réduite pour donner le composé **(3)** (53,31 g, 60 %) sous forme d'un solide blanc.
RMN ¹H (300MHz, CDCl₃) : 2,25 (s,CH3), 2,31 (s,CH3), 2,45 (t,CH2), 2,71 (t,CH2), 3,67 (s,CH3), 5,30 (s,CH2), 7,40 (m,5H), 8,60 (singulet large, NH).
RP-HPLC :
HP Hypersil BDS-C C18, 125x4mm, 25°C
Solvants avec 0,1% de TFA : acétonitrile (ACN)-Eau : de 1 à 100% de ACN pendant 10 min puis 2 min avec 100% de ACN
Débit : 1mL/min, détection à 220 nm
Echantillon : 1mg/1,5ml de ACN
Rt : 8,53 min (>98%)

### PREPARATION 2

### Préparation du composé de formule (VIII.1) :

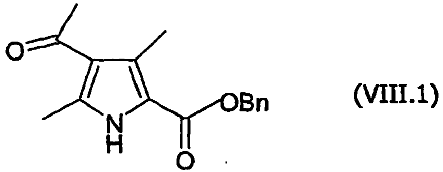

Un ballon Keller de 2,5 L équipé d'un réfrigérant à reflux/tête de distillation, d'un thermomètre, d'une ampoule à brome et d'un conduit à argon est chargé avec une solution de 4-acétyl-3,5-diméthyl-pyrrole-2-carboxylate d'éthyl (produit commercial, Alpha Aesar,Karlsruhe,DE, product n°. A 17365) (146,00 g, 0,70 mol) dans l'alcool benzylique (1,00 L) et chauffé à 120°C, ce qui conduit au retrait azéotropique de quantités mineures d'eau. Le mélange est ensuite chauffé à 190°C. L'ampoule à brome est chargée avec une solution préparée séparément de sodium (2 g) dans l'alcool benzylique (20 mL). Cette solution est ajoutée par portions de 5 mL, ce qui conduit à un reflux vigoureux du mélange réactionnel. Le méthanol et l'éthanol résultants sont retirés de manière semi-continue par distillation. La conversion est suivie par HPLC. Le mélange réactionnel est mis à refroidir à 150°C puis transféré sur un mélange de méthanol (0,96 L), d'eau (0,66 L) et d'acide acétique (12 mL). Le mélange est refroidi à -10°C et agité encore à cette température pendant 1,5 h. Le produit précipité est isolé par filtration. Le produit est séché sous pression réduite pour donner le composé **(VIII.1)** (124,40 G, 65 %) sous forme d'un solide blanc cassé.
RMN ¹H (300MHz, CDCl₃) : 2,34 (s,CH3), 2,40 (s,CH3), 2,52 (s,CH3), 5,23 (s,CH2), 7,85 (m,5H), 9,55 (singulet large, NH)

### PREPARATION 3

### Préparation du composé de formule (VI.1) :

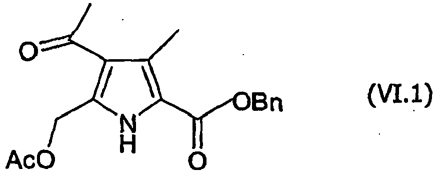

Un ballon Keller de 2,5 L équipé d'un réfrigérant à reflux, d'un thermomètre, d'une ampoule à brome et d'un conduit à argon est chargé avec du composé **(VIII.1)** (66,84 g, 0,25 mol), de l'acide acétique (1,25 L) et de l'acétate de sodium (73,90 g, 1,51 mol). Pour obtenir une solution limpide, le mélange est chauffé à ~35°C puis mis à refroidir à la température ambiante. En 2 h, du chlorure de sulfuryle (32,4 mL, 0,40 mol) est ajouté tandis que la réaction est contrôlée soigneusement vers la fin de l'addition, pour minimiser la formation de sous-produit par sur-réaction. A la température ambiante, des quantités supplémentaires d'acétate de sodium (50,0 g) sont ajoutées et le mélange est agité encore à la température ambiante pendant une nuit. De l'eau (500 mL) est ajoutée pour donner une solution limpide. Après addition d'un mélange eau-méthanol 9-1 (4,5 L), le mélange réactionnel est agité encore à la température ambiante pendant 1 h avec précipitation du produit. Le produit est isolé par filtration et dissous par chauffage à reflux dans l'acétate d'éthyle (220 mL). Le mélange biphasique est retiré du bain d'huile et du méthanol (200 mL) est ajouté sous agitation. Après agitation encore pendant 1 h à la température ambiante, le produit commence à cristalliser. Des quantités supplémentaires de méthanol (500 mL) sont ajoutées et le mélange agité est refroidi à -10°C. Le produit est isolé par filtration. Le produit est séché sous pression réduite pour donner le composé **(VI.1)** (37,14 g, 45 %) sous forme d'un solide blanc.
RMN ¹H (300MHz, CDCl₃) : 2,07 (s,CH3), 2,41 (s,CH3), 2,53 (s,CH3), 5,27 (s,CH2), 5,31 (s,CH2), 7,32 (m, 5H), 9,40 (singulet large, NH).
RP-HPLC :
HP Hypersil BDS-C C18, 125x4mm, 25°C
Solvants avec 0,1% de TFA : acétonitrile (ACN)-Eau: de 1 à 100% de ACN pendant 10 min puis 2 min avec 100% de ACN
Débit : 1mL/min, détection à 220 nm
Echantillon : 1mg/1,5ml de ACN
Rt : 7,89 min (>94%)

### PREPARATION 4

### Préparation du composé de formule (7) :

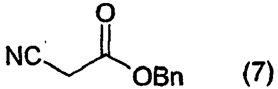

### a) Préparation du composé de formule (9) :

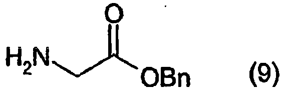

Un ballon Keller de 2,5 L équipé d'un réfrigérant à reflux, d'un thermomètre, d'une ampoule à brome et d'un conduit à argon est chargé avec de la glycine (80,00 g, 1,07 mol), de l'alcool benzylique (700 mL) et de l'acide p-toluènesulfonique monohydraté (241 g, 1,27 mol). Le mélange blanc épais est chauffé à 100°C, ce qui conduit à la formation d'une solution limpide. Le mélange est agité encore à 100°C pendant 5 h. Le mélange est mis à refroidir à la température ambiante. Du diéthyléther (4 L) est ajouté lentement ce qui conduit à la précipitation du produit. Le produit est isolé par filtration, lavé à l'éther (3 x 0,3 L) et séché sous pression réduite à 60°C. Du fait de la conversion incomplète, le solide blanc est repris dans le toluène (2,3 L), de l'alcool benzylique (0,3 L) et de l'acide p-toluènesulfonique monohydraté (20 g) est ajouté. Le mélange est chauffé à reflux pendant 4 h tandis que l'eau est retirée en continu par le biais d'un appareil de Dean-Stark. Le mélange est mis à refroidir à la température ambiante. Du diéthyléther (1 L) est ajouté lentement et le mélange est refroidi à 0°C, ce qui conduit à la précipitation du produit. Le produit est isolé par filtration, lavé à l'éther (3 x 0,3 L) et séché sous pression réduite à 60°C pour donner le composé (9) (303,20 g, 84 %) sous forme d'un produit cristallin blanc.
RMN ¹H (300MHz, DMSO-D₆) : 2,28 (s,CH3), 3,90 (s,CH2), 5,25 (s,CH2), 7,15 et 7,39 (AB, 4H), 7,37 (m,5H).

### b) Préparation du composé de formule (8) :

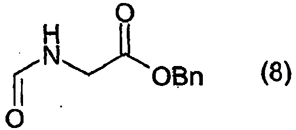

Un ballon Keller de 2,5 L équipé d'un réfrigérant à reflux, d'un thermomètre, d'une ampoule à brome et d'un conduit à argon est chargé avec du formiate de méthyle (700 mL), du composé **(9)** (303,0 g, 898,0 mmol) et de la triéthylamine (137 mL, 1 mol). Le mélange est chauffé à reflux pendant 22 h. La conversion est suivie par HPLC. Le mélange hétérogène est concentré sous pression réduite pour donner une huile (429 g). Le produit est dissous dans le dichlorométhane (1,5 L), lavé avec du bicarbonate (2 x 0,5 L) et de l'eau (0,5 L). La phase aqueuse combinée est ré-extraite avec du dichlorométhane (0,5 L). La phase organique combinée est séchée (Na₂SO₄), filtrée et concentrée sous pression réduite (40 mbar, 45°C, 1 h) pour donner le composé **(8)** (149,2 g, 86 %) sous forme d'une huile jaune-orange.
RMN ¹H (300MHz, CDCl₃) : 4,12 (d,CH2), 5,19 (s,CH2), 6,33 (singulet large, NH), 8,23 (s, CHO).

### c) préparation du composé de formule (7) :

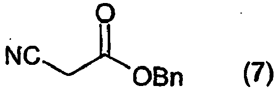

Un ballon Keller de 2,5 L équipé d'un réfrigérant à reflux, d'un thermomètre, d'une ampoule à brome et d'un conduit à argon est chargé avec le composé **(8)** (129,7 g, 671,3 mmol) et du dichlorométhane (1 L). Le mélange est refroidi à 0°C. De la triéthylamine (234 mL, 1678 mol) est ajoutée pour donner une solution jaune. POCl₃ (102,9 g, 671,3 mmol) est ajouté en 50 min tandis que la température est maintenue entre 0 et 5°C. Le mélange est agité encore pendant 2 h tandis qu'il est chauffé à la température ambiante. Une solution de K₂CO₃ (134 g) dans l'eau (600 mL) est ajoutée lentement et avec précaution par petites portions entre 25 et 30°C. Après l'addition complète, le mélange est agité encore pendant 1 h. De l'eau (1 L) est ajoutée et les phases sont séparées. La phase organique est lavée avec de l'eau (0,2 L). La phase aqueuse combinée est ré-extraite avec du dichlorométhane (0,5 L). La phase organique combinée est séchée (Na₂SO₄), filtrée et concentrée sous pression réduite pour donner une huile brune (180 g). La chromatographie est réalisée sur silice (500 g), avec élution avec du dichlorométhane. Les fractions contenant le produit sont regroupées et concentrées sous pression réduite pour donner une huile jaune-orange. Le stockage à-10°C permet la cristallisation du composé (7) (101,9 g, 87 %) pour former un produit stable.
RMN ¹H (300MHz, CDCl₃) : 4,22 (s,CH2), 5,23 (s,CH2), 7,38 (s,5H).

### PREPARATION 5

### Préparation du composé de formule (IX.1) :

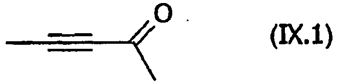

Un ballon Keller de 4,5 L équipé d'un réfrigérant à reflux, d'un thermomètre, d'une ampoule à brome et d'un conduit à argon est chargé avec du trichlorure d'aluminium (297,00 g, 2,23 mol) et du dichlorométhane (2,3 L) et refroidi à 0°C. Une solution de triméthylsillylpropyne (250,00 g, 2,23 mol) et de chlorure d'acétyle (0,16 L, 2,23 mol) dans le dichlorométhane (0,4 L) a été ajoutée à la suspension jaune clair en 1,5 h en maintenant la température entre 0 et 5°C. La solution brune avec une certaine quantité de sel précipité est mise à chauffer à la température ambiante. La solution brun rouge résultante est versée dans la glace/eau (2 L). Les couches sont séparées et la phase aqueuse extraite avec du dichlorométhane (0,5 L). La phase organique combinée est lavée avec de l'eau (0,5 L), séchée (Na₂SO₄), filtrée et concentrée sous pression réduite pour donner un liquide vert-noir (558 g). Le produit est distillé à 180 mbar pour donner une fraction (~160 g) bouillant entre 64 et 70°C. Ce produit est distillé de nouveau à 210 mbar pour donner le composé **(IX.1)** (90,30 g, 49 %) sous forme d'un liquide incolore présentant un point d'ébullition entre 81 et 85°C.
RMN ¹H (300MHz, CDCl₃) : 2,02 (s,CH3), 2,31 (s,CH3).

### PREPARATION 6

### Préparation du composé de formule (VIIa) :

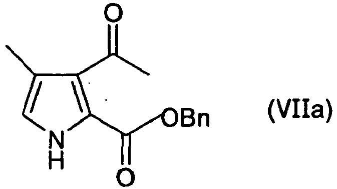

Un ballon Keller de 1 L équipé d'un réfrigérant à reflux, d'un thermomètre, d'une ampoule à brome et d'un conduit à argon est chargé successivement avec le composé **(7)** (101,90 g, 0,58 mmol), du dioxane (0,5 L) et le composé **IX-1** (52,53 g, 0,64 mol). Par addition de méthyldiphénylphosphine (38,4 g, 0,19 mol), la réaction devient très exothermique.

Le mélange réactionnel devient foncé et est chauffé à 100°C pendant 1 h. La conversion est suivie par HPLC. Les produits volatils sont retirés sous pression réduite. L'huile brune brute (209 g) est purifiée par chromatographie sur silice (2,1 kg) en éluant avec un mélange toluène/acétate d'éthyle (8/1). Les fractions contenant le produit pur sont combinées et les produits volatils sont retirés sous pression réduite pour donner le composé **(VIIa)** (73,49 g, 49 %) sous forme d'une huile sirupeuse brun clair.
RMN ¹H (300MHz, CDCl₃): 2,13 (s,CH3), 2,54 (s,CH3), 5,32 8s,CH2), 6,68 (d, CH), 7,38 (m,5H), 9,20 (singulet large, NH).
RP-HPLC :
HP Hypersil BDS-C C18, 125x4mm, 25°C
Solvants avec 0,1% de TFA : acétonitrile (ACN)-Eau : de 1 à 100% de ACN pendant 10 min puis 2 min avec 100% de ACN
Débit : 1 mL/min, détection à 220 nm
Echantillon : 1mg/1,5ml de ACN
Rt : 7,55 min (>91%)

### PREPARATION 7

### Préparation du pyrrométhane de formule (2) :

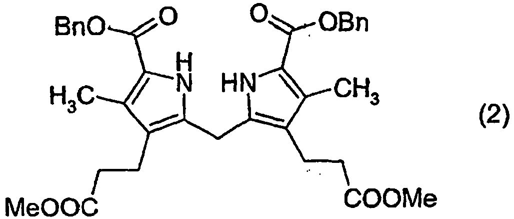

Un ballon Keller de 4,5 L équipé d'un réfrigérant à reflux, d'un thermomètre, d'une ampoule à brome et d'un conduit à argon est chargé avec le composé **(3)** (52,0 g, 165,0 mmol) et du diéthyléther (1,5 L). Une solution de brome (11,0 mL, 214,0 mmol) dans le diéthyléther (0,5 L) fraîchement préparée est ajoutée goutte à goutte en 20 min à la température ambiante pour produire une solution orange-brun. La conversion est suivie par HPLC. Si nécessaire, des quantités supplémentaires de brome sont ajoutées. Le mélange est agité encore à la température ambiante. Les produits volatils sont retirés sous pression réduite et le résidu gris brun est dissous dans le méthanol (364 mL). La solution est chauffée à ~50°C jusqu'à ce que la conversion complète soit obtenue, (déterminée par HPLC après environ 11 h). Le mélange réactionnel sombre est concentré sous pression réduite jusqu'à ce que le produit commence à cristalliser. Le produit précipité est isolé par filtration et lavé avec du méthanol (0,2 L). Le produit brut est recristallisé par mise en suspension dans le diéthyléther (0,6 L) et chauffage à reflux, tandis que de l'heptane (1,8 L) est ajouté et le chauffage est continué pour maintenir le mélange à reflux pendant 15 min supplémentaires. Le mélange est mis à refroidir à la température ambiante et le produit est isolé par filtration. Le produit est séché pour donner le composé (2) (31,70 g, 63 %) sous forme d'une poudre gris clair.
RMN ¹H (300MHz, CDCl₃) : 2,21 (s, deux CH3), 2,43 (t, deux CH2), 2,68 (t, deux CH2), 3,50 (s, deux CH3), 3,89 (s,CH2), 5,17(s, deux CH2), 7,20 (m, 10H), 9,00 (singulet large, deux NH)

### PREPARATION 8

### Préparation du pyrrométhane de formule (1) :

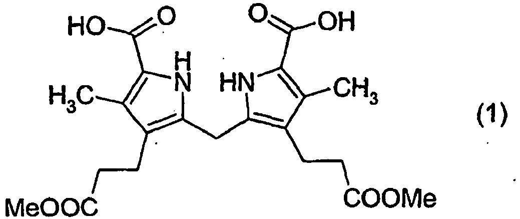

Un appareil d'hydrogénation basse pression est chargé avec le composé **(2)** (30,7 g, 49,9 mmol), du THF (400 mL) et du catalyseur Pd/C à 10 % (1,5 g, A027). L'hydrogénation a lieu à la température ambiante sous une atmosphère de pression d'hydrogène en 3 h. De l'ammoniac 2N (0,1 L) est ajouté au mélange réactionnel et le catalyseur est retiré par filtration. Le filtrat est ajusté à pH ~7 par addition d'acide acétique (60 mL). Le solvant est retiré sous pression réduite. Le produit précipité est isolé par filtration pour donner après séchage le composé **(1)** (21,7 g, quant.) sous forme d'une poudre blanche.
RMN ¹H (300MHz, DMSO-D6) : 2,18 (s, deux CH3), 2,20 (t, deux CH2), 2,59 (t, deux CH2), 3,60 (s, deux CH3), 3,82 (s,CH2)

### PREPARATION 9

### Préparation du pyrrométhane de formule (II.1) :

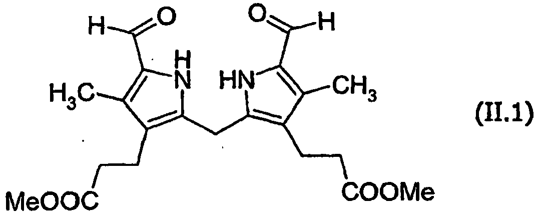

Un ballon Keller de 1 L équipé d'un réfrigérant à reflux, d'un thermomètre, d'une ampoule à brome et d'un conduit à argon est chargé avec de l'acide trifluoroacétique (190 mL) et refroidi à 0°C. Le composé **(1)** (20,0 g, 46,0 mmol) est ajouté par petites portions en 10 min à 0°C. Le mélange est agité encore à 0°C pendant 1 h. La conversion est suivie par HPLC. De l'orthoformiate de triméthyle (57 mL) est ajouté goutte à goutte en 30 min tandis que la température est maintenue entre 0 et 5°C. Le mélange réactionnel est agité encore pendant 1 h à 0°C puis versé sur de l'eau (1,7 L). Le mélange est agité vigoureusement pendant 10 min. Le produit brut précipité est isolé par filtration et lavé à l'eau (0,3 L) sous forme d'une poudre orange. Le produit brut est trituré dans l'éthanol (0,2 L) et l'ammoniac (0,4 L). Le mélange est agité pendant 30 min à la température ambiante et le produit est isolé par filtration et lavé à l'eau (0,3 L) sous forme d'une poudre jaune foncé. Le produit est chauffé à reflux dans le méthanol (0,4 L) pendant 10 min. Le mélange est mis à refroidir à la température ambiante et le produit est isolé par filtration et lavé avec du méthanol froid (0,1 L). Le produit est séché sous pression réduite pour donner le composé **(II.1)** (15,30 g, 83 %) sous forme d'une poudre jaune clair.
RMN ¹H (300MHz, CDCl₃) : 2,30 (s, deux CH3), 2,53 (t, deux CH2), 2,81 (t, deux CH2), 3,72 (s, deux CH3), 4,06 (s, CH2), 9,46 (s, deux CHO), 10,43 (singulet large, deux NH).
RP-HPLC :
HP Hypersil BDS-C C18, 125x4mm, 25°C
Solvants avec 0,1% de TFA : acétonitrile (ACN)-Eau : de 1 à 100% de ACN pendant 10 min puis 2 min avec 100% de ACN
Débit : 1 mL/min, détection à 220 nm
Echantillon : 1mg/1,5ml de ACN
Rt : 6,78 min (>96%)

### PREPARATION 10

### Préparation du pyrrométhane de formule (Va) :

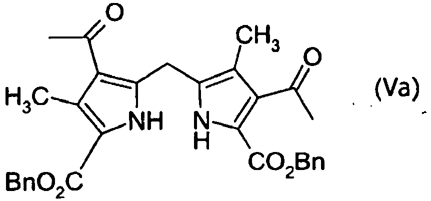

- Un ballon Keller de 2,5 L équipé d'un réfrigérant à reflux, d'un thermomètre, d'une ampoule à brome et d'un conduit à argon est chargé avec le composé **(VIIa)** (50,0 g, 194,4 mmol), le composé **(VI.1)** (51,3 g, 155,6 mmol) et du dichloroéthane (1,1 L). Le mélange est chauffé à 40°C pour donner une solution rouge-orange. De l'éthérate de HBF₄ (2,35 mL (54 %), 9,3 mmol) est ajouté et le mélange est chauffé rapidement à 90°C. La conversion est suivie par HPLC. Au bout de 1 h, le mélange est refroidi rapidement à la température ambiante et versé sur une solution de bicarbonate saturé (0,5 L). Les couches sont séparées et la phase aqueuse est extraite avec du dichloroéthane (0,5 L). Les extraits organiques combinés sont séchés (Na₂SO₄), filtrés, agités avec du Norrit C (2 g), filtrés et concentrés totalement sous pression réduite pour donner un sirop collant brun (96,5 g). Le produit brut est dissous dans du méthanol (0,3 L), concentré sous pression réduite et dissous de nouveau dans le méthanol (150 mL). Des cristaux de germination sont ajoutés et le mélange est laissé au repos pendant 15 h à la température ambiante tandis que le produit cristallise. Le surnageant est retiré et les cristaux (fraction K1, 34,3 g) sont lavés avec du méthanol. Les fractions de méthanol combinées sont concentrées totalement sous pression réduite et chromatographiées sur silice (420 g) avec élution avec de l'hexane/acétate d'éthyle (2/1). Les fractions contenant le produit sont regroupées et concentrées sous pression réduite. Le produit est recristallisé comme précédemment dans le méthanol pour donner une fraction K2 (16,7 g). Le surnageant est de nouveau chromatographié sur silice (400 g) avec élution avec de l'hexane/acétate d'éthyle (2/1). Les fractions contenant le produit sont regroupées et concentrées sous pression réduite. Le produit est recristallisé comme précédemment dans le méthanol pour donner une fraction K3 (3,7 g). Les fractions de produit (K1 - K3) sont combinées, dissoutes dans le toluène et totalement concentrées sous pression réduite. Après séchage sous pression réduite à 50°C pendant 1 h, le composé **(Va)** (54,7 g, 68 %) est récupéré sous forme de cristaux blanc cassé.
RMN ¹H (300MHz, CDCl₃) : 2,09 (2,09,CH3), 2,49 (s, CH3), 2,50 (s,CH3), 2,58 (s,CH3), 4,04 (s, CH2), 5,27 (s,CH2), 5,29 (s, CH2), 7,35 (m, 10H), 10,5 (singulet large, NH).

### PREPARATION 11

### Préparation du pyrrométhane de formule (IVa) :

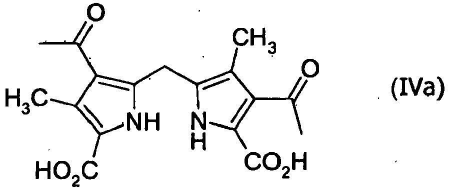

Un appareil d'hydrogénation basse pression est chargé avec le composé **(Va)** (54,3 g, 103,1 mmol), du tétrahydrofurane (700 mL), de la triéthylamine (20,8 g, 206,2 mmol) et du catalyseur Pd/C à 10 % (2,75 g). L'hydrogénation a lieu à la température ambiante sous une atmosphère de pression d'hydrogène en 3 h. Le catalyseur est retiré par filtration. Le filtrat est concentré sous pression réduite. Après séchage sous pression réduite à 45°C pendant 0,5 h, le composé **(IVa)** (54,7 g, quant.) est récupéré sous forme d'une mousse blanc cassé sous forme d'un sel de mono-triéthylamine contenant des quantités résiduelles de toluène et de THF.
RMN ¹H (300MHz, DMSO-D6) : 1,97 (s,CH3), 2,38 (s, CH3), 2,52 (s, deux CH3), 4,14 (s, CH2)

### PREPARATION 12

### Préparation du pyrrométhane de formule (IIIa) :

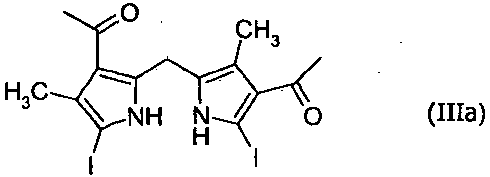

Un ballon Keller de 2,5 L équipé d'un réfrigérant à reflux, d'un thermomètre, d'une ampoule à brome et d'un conduit à argon est chargé avec NaHCO₃ solide (55,6 g, 662,0 mmol), de l'eau (900 mL) et de l'éthanol (300 mL). Une solution du composé **(IVa)** (54,3 g, 101,9 mmol) dans l'éthanol (300 mL) est ajoutée. A la température ambiante, une solution d'iode (64,7 g, 254,9 mmol) dans l'éthanol (400 mL) est ajoutée pour donner un mélange hétérogène brun. Un certain moussage et une exothermie atténuée sont observés. La conversion est suivie par HPLC. Le mélange réactionnel est agité encore à la température ambiante pendant 5 h. Le mélange réactionnel est dilué à l'eau (0,1 L) et le produit précipité est isolé par filtration. Le précipité est lavé avec de l'eau (3 x 0,1 L), de l'éthanol (2 x 0,1 L) et de l'éther (2 x 0,1 L). Après séchage des cristaux de produit sous pression réduite à 60°C; le composé (**III**.**a**) (48,1 g, 92 %) est récupéré sous forme de cristaux rouge clair.
RMN ¹H (300MHz, DMSO-D₆) : 2,06 (s, CH3), 2,15 (s, CH3), 2,33 (s, CH39, 4,08 (s, CH2)
RP-HPLC :
HP Hypersil BDS-C C18, 125x4mm, 25°C
Solvants avec 0,1% de TFA : acétonitrile (ACN)-Eau : de 1 à 100% de ACN pendant 10 min puis 2 min avec 100% de ACN
Débit : 1 mL/min, détection à 220 nm
Echantillon : 1mg/1,5ml de ACN
Rt : 8,13 min (>92%)

### PREPARATION 13

### Préparation de la porphyrine de formule (Ia.1) :

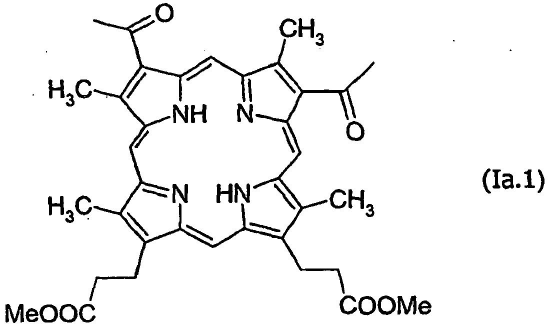

Un ballon Keller de 2,5 L équipé d'un réfrigérant à reflux, d'un thermomètre, d'une ampoule à brome et d'un conduit à argon est chargé avec de l'anhydride d'acide acétique (290 mL), de l'acide acétique (1,8 L) et de l'acide trifluorométhanesulfonique (4,95 mL, 56,77 mmol). A la température ambiante, une solution sensiblement homogène du composé **(II.1)** (12,00 g, 29,82 mmol) et du composé **(IIIa)** (14,48 g, 28,40 mmol) dans l'acide acétique (400 mL) est ajoutée en 5 min ce qui conduit à une solution rouge sang. Aucune exothermie n'est observée. Le mélange est agité encore à la température ambiante pendant 1 h avec la formation d'un certain précipité. La conversion est suivie par HPLC. Une solution de NaOAc (9,4 g) dans l'acide acétique (100 mL) est ajoutée pour donner une solution brun foncé. Au bout de 10 min, les produits volatils sont retirés sous pression réduite et séchés sous pression réduite pendant 1,5 h à 50°C. Le résidu sombre est repris dans le dichlorométhane (300 mL), l'eau (500 mL) sans mélange vigoureux. La couche organique est séparée et la phase aqueuse est extraite avec du dichlorométhane (0,3 L). La phase organique combinée est séchée (Na₂SO₄), filtrée et concentrée pour donner un produit cristallin noir (31,3 g). Le mélange est dissous dans le dichlorométhane et appliqué sur une colonne de gel de silice (1 kg) garni de dichlorométhane/acétone (95/5). Le produit est élué avec un gradient de 95/5 à 90/10. Les fractions contenant le produit sont regroupées et concentrées totalement sous pression réduite. Le composé **(Ia.1)** (9,97 g, 55 %) est récupéré sous forme de cristaux violet-noir.
RMN ¹H (300MHz, CDCl3): 3,15 (deux t, deux CH2), 3,17 (s,CH3), 3,25 (s,CH3), 3,39. (s,CH3), 3,50 (s,CH3), 3,60 (s, CH3), 3,64 (s,CH3), 3,65 (s,CH3), 3,71 (s,CH3), 4,22 (deux t, deux CH2), 9,50 (s, CH), 9,59 (s, CH), 10,43 (s, CH), 10,46 (s, CH).
RP-HPLC :
HP Hypersil BDS-C C18,125x4mm, 25°C
Solvants avec 0,1% de TFA : acétonitrile (ACN)-Eau : de 1 à 100% de ACN pendant 10 min puis 3 min avec 100% de ACN
Débit : 1mL/min, détection à 220 nm
Echantillon : 1mg/1,5ml de ACN
Rt : 11,66 min (>93%)

### PREPARATION 14

### Préparation de la porphyrine de formule (Ib.1) :

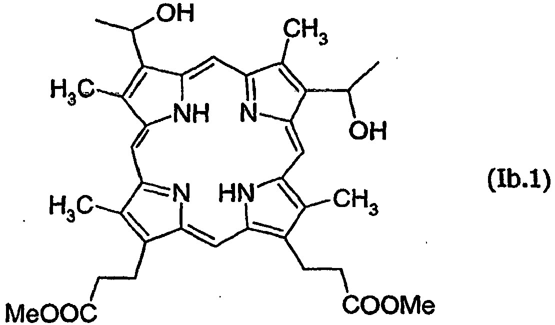

Un ballon Keller de 1 L équipé d'un réfrigérant à reflux, d'un thermomètre, d'une ampoule à brome et d'un conduit à argon est chargé avec le composé **(Ia.1)** (9,86 g, 15,84 mmol), du dichlorométhane (500 mL) et du méthanol (24 mL). NaBH₄ (3,00 g, 79,32 mmol) est ajouté par portion au mélange brun rouge. Un certain moussage est observé. La réaction est suivie étroitement par HPLC. Au bout de 80 min, le mélange est versé sur un mélange d'eau (500 mL) et de HCl 4N (80 mL). Un dégagement gazeux est observé. Le mélange est neutralisé par addition de NaHCO₃ solide. Les couches sont séparées et la phase aqueuse est extraite avec du dichlorométhane (2 x 300 mL). Les extraits organiques combinés sont séchés (Na₂SO₄), filtrés et concentrés sous pression réduite. Après séchage sous pression réduite à 50°C pendant 0,5 h, le composé **(Ib.1)** sous la forme d'un mélange de deux stéréoisomères (9,58 g, 96 %) est récupéré sous forme de cristaux violet-noir.
RMN ¹H (300MHz, CDCl3): 1,92(m,6H, deux CH3), 3,16 (m,4H, deux CH2), 3,28, 3,30, 3,33 and 3,35 (4s, 6H, deux CH3), 3,43 (s,6H, deux CH3), 3,66 (s,6H, deux CH3), 4,20 (m,4H, deux CH2), 6,05 (m, 2H, deux CH), 9,73, 9,74, 9,75, 9,76, 10,00, 10,02, 10,08 et 10,10 (8s, total 4H, quatre CH).
RP-HPLC :
HP Hypersil BDS-C C18, 125x4nim, 25°C
Solvants avec 0,1% de TFA : acétonitrile (ACN)-Eau : de 1 à 100% de ACN pendant 10 min puis 3 min avec 100% de ACN
Débit : 1mL/min, détection à 220 nm
Echantillon : 1mg/1,5ml de ACN
Rt : 7,90 min., 51 % et 8,01 min., 49%

### PREPARATION 15

### Préparation de la porphyrine de formule (Ic.1) :

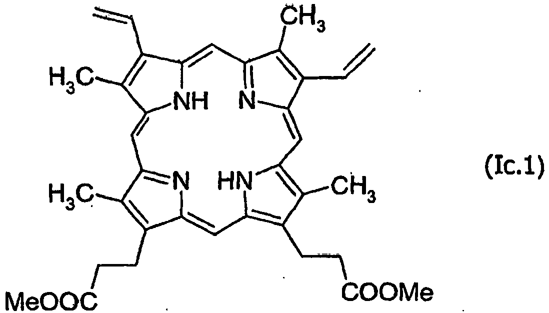

Un ballon de Keller de 1 L équipé d'un réfrigérant à reflux, d'un thermomètre, d'une ampoule à brome et d'un conduit à argon est chargé avec le composé **(Ib.1)** (9,48 g, 15,12 mmol) et du DMF (400 mL) et le mélange est dégazé avec de l'argon. Du chlorure de benzoyle (45,0 mL, 387,9 mmol) est ajouté et le mélange est chauffé rapidement à 100°C. Le mélange est agité encore à 100°C pendant 1 h. La conversion est suivie par HPLC. Le mélange réactionnel est refroidi rapidement et les produits volatils sont retirés sous pression réduite. Le résidu est dissous dans le dichlorométhane (0,3 L) et agité vigoureusement avec un mélange d'eau/méthanol (0,3 L). Les couches sont séparées et la phase aqueuse est extraite avec du dichlorométhane (2 x 0,2 L). Les extraits organiques combinés sont lavés avec du bicarbonate (0,3 L), séchés (Na₂SO₄) et filtrés. Le filtrat est traité avec de la silice (20 g) et filtré. Du méthanol (50 mL) est ajouté et le mélange est ensuite concentré sous pression réduite tandis qu'une cristallisation se produit vers la fin pour donner un produit violet-noir (20 g). Le produit est trituré avec du méthanol à 50°C pendant 0,5 h. Après refroidissement à la température ambiante, du chloroforme (2 mL) est ajouté et le produit est isolé par filtration. Après séchage sous pression réduite à 50°C pendant 15 h, le composé **(Ic.1)** (6,34 g, 77 %) est récupéré sous forme de cristaux brillants violet-noir.
RMN ¹H (300MHz, CDC13): 3,23 (t, deux CH3), 3,52 (s,CH3), 3,54 (s,CH3), 3,58 (s,CH3), 3,64 (s,CH3), 3,64 (s,CH3), 3,65 (s, CH3), 3,66 (s,CH3), 4,32 (t, deux CH2), 6,11-6,34 (m, 4H, deux CH2=), 8,10-8,23 (m, 2H, deux CH=), 9,85, 9,86, 9,97 and 9,98 (4s,4CH).
RP-HPLC :
HP Hypersil BDS-C C18, 125x4mm, 25°C
Solvants avec 0,1% de TFA : acétonitrile (ACN)-Eau : de 1 à 100% de ACN pendant 10 min puis 6 min avec 100% de ACN
Débit : 1mL/min, détection à 220 nm
Echantillon : 1mg/1,5ml de ACN
Rt : 12,43 min (>97%)

### PREPARATION 16

### Préparation de la porphyrine de formule (IC.2, 2Na) :

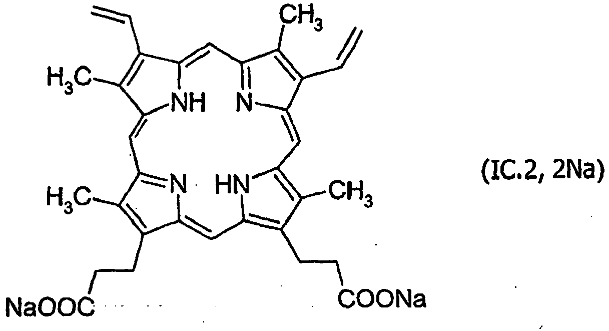

Un ballon Keller de 2,5 L équipé d'un réfrigérant à reflux, d'un thermomètre, d'une ampoule à brome et d'un conduit à argon est chargé avec le composé **(Ic.1)** (6,30 g, 10,67 mmol) et du dichlorométhane (200 mL). Le produit est dissous par chauffage à 40°C. A 40°C, successivement, du méthanol (400 mL) est ajouté puis NaOH 4N (200 mL). La formation d'un précipité est observée. Le mélange est chauffé à reflux. La conversion est suivie par HPLC. Les produits volatils organiques sont retirés sous pression réduite. La suspension est filtrée sur un filtre en fibre de verre. Le produit est lavé avec de l'eau (3 x 0,1 L), du méthanol (3 x 30 mL) et du diéthyléther (2 x 30 mL). Après séchage sous pression réduite à 70°C pendant 2 h puis à 40°C pendant 15 h, le composé **(Ic.2, 2Na)** (6,06 g, 94 %) est récupéré sous forme d'un produit solide violet-noir.
RMN ¹H (300MHz, TFA-D₁) : 3,45 (deux t, deux CH2), 3,82 (s,CH3), 3,85 (s,CH3), , 3,88 (s,CH3), 3,91 (s,CH39, 4,73 (deux t, deux CH2), 6,43-6,70 (m, 4H, deux CH2=), 8,28-8,40 (m, 2H, deux CH=), 11,08, 11,11, 11,15, and 11,27 (4s,4CH).
RP-HPLC :
HP Hypersil BDS-C C18, 125x4mm, 25°C.
Solvants: acétonitrile (ACN) avec 0,1% de TFA -Eau avec 0,1% de TFA: de 1 à 100% de ACN pendant 10 min puis 6 min avec 100% de ACN
Débit : 1mL/min, détection à 220 nm
Echantilion : 0,1mg/1,5ml de AcOH/DMF
Rt : 9,85 min (>98%)

### Analyse élémentaire

| | Théorie pour C34.H32.N4.04.Na2 (MW 606.63) | Résultat |
|---|---|---|
| C | 67,32 +/-0,3% m/m | 64,91 % m/m |
| H | 5,32 +/-0,3% m/m | 5,38 % m/m |
| N | 9,24 +/-0,3% m/m | 8,94 % m/m |
| Eau | | 3,56 % m/m |
| Na | 7,58 m/m | 7,15 % m/m |

Avec des mesures ajustées après ajout de 1,24 mol d'eau (quantité d'eau mesurée par titrage Karl-Fisher) par mol de produit.

| | Théorie pour C34.H32.N4.04.Na2.H2O1,24 (MW 636.54) | Résultat |
|---|---|---|
| C | 64,93 +/-0,3% m/m | 64,91 % m/m |
| H | 5,28 +/-0,3% m/m | 5,38 % m/m |
| N | 8,91 +/-0,3% m/m | 8,94 % m/m |
| Eau | 3,56 m/m | 3,56 % m/m |
| Na | 7,31 m/m | 7,15 % m/m |

## Revendications

1. Procédé de préparation d'une porphyrine de formule (I), éventuellement sous la forme d'un sel : 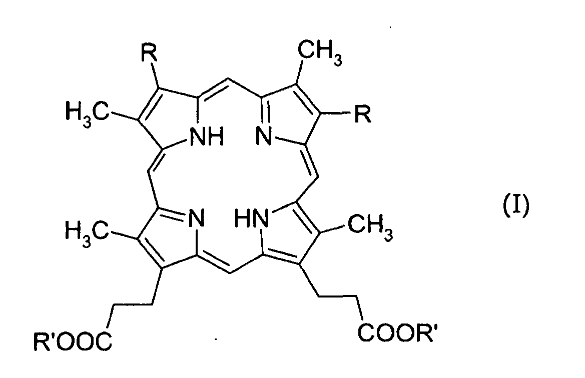 dans laquelle :
- R représente un atome d'hydrogène ou un groupe choisi parmi : -CH=CH₂, -CH₂-CH₃, - CH(OH)CH₃, -C(O)CH₃, -CH₂CH₂COOR'a avec R'a qui représente un atome d'hydrogène ou un groupe méthyle, éthyle, n-propyle ou i-propyle,
- R' représente un atome d'hydrogène ou un groupe R'b choisi parmi méthyle, éthyle, n-propyle ou i-propyle,
comprenant :
- une étape de condensation, en milieu acide, entre un dipyrrométhane de formule (II) : 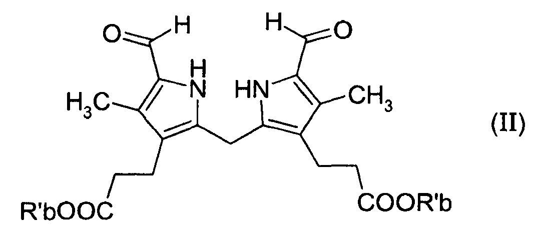 dans laquelle R'b est tel que défini précédemment pour (I),
et un dipyrrométhane de formule (III) : 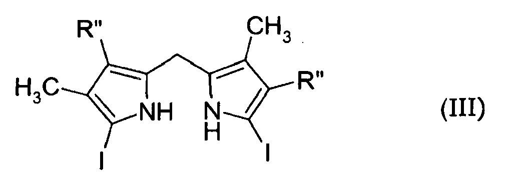
dans laquelle R" est identique à R tel que défini précédemment pour (I) ou est un groupe précurseur de R, pour former la porphyrine de formule (I') : 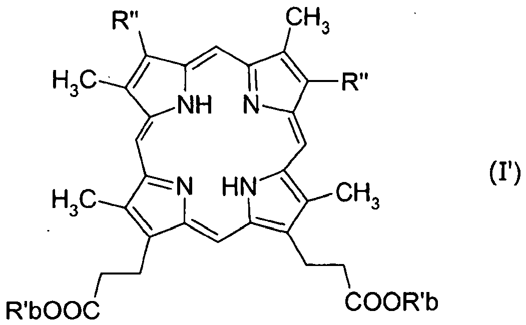 dans laquelle R" et R'b sont tels que définis précédemment pour (II) et (III),
suivie :
- lorsque R" est un groupe précurseur de R, de la transformation des groupes R" en R,
- lorsque R'=H, d'une élimination des groupes R'b pour former les groupes -COOH, éventuellement sous forme de sel.

2. Un procédé selon la revendication 1, dans laquelle R" est un atome d'hydrogène ou un groupe choisi parmi: -CH=CH₂, -CH₂-CH₃, -CH(OH)CH₃, -C(O)CH₃, -CH₂CH₂OH, - CH₂CH₂Cl, -CH₂CH₂OC(O)CH₃ -CH₂CH₂COOR'a avec R'a qui représente un atome d'hydrogène ou un groupe méthyle, éthyle, n-propyle ou i-propyle.

3. Procédé selon la revendication 1 ou 2 **caractérisé en ce qu'**il est mis en oeuvre pour la préparation d'un composé de formule (I) dans laquelle R représente un atome d'hydrogène ou un groupe choisi parmi : -CH₂-CH₃, -CH(OH)CH₃, -C(O)CH₃, -CH₂CH₂COOR'a avec R'a qui représente un atome d'hydrogène ou un groupe méthyle, éthyle, n-propyle ou i-propyle.

4. Procédé selon la revendication 1 ou 2 **caractérisé en ce qu'**il est mis en oeuvre pour la préparation d'un composé de formule (I) dans laquelle R représente un groupe -CH=CH₂, comprenant :
une étape de condensation, en milieu acide, entre un dipyrrométhane de formule (II) : 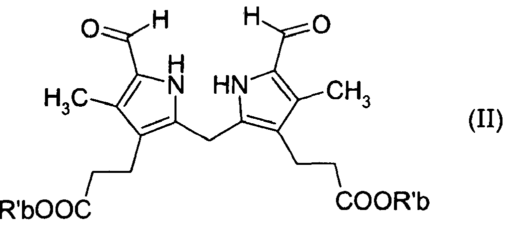 dans laquelle R'b est tel que défini précédemment pour (I),
et un dipyrrométhane de formule (III) : 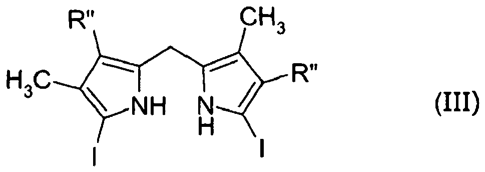
dans laquelle R" est un groupe précurseur de R, suivie de la transformation des groupes R" en R,
et lorsque R'=H, de l'élimination des groupes R'b pour obtenir les groupes -COOH, éventuellement sous forme de sel.

5. Procédé selon la revendication 4 **caractérisé en ce que** R" est un groupe -CH₂CH₂OH, - CH₂CH₂Cl, -CH₂CH₂OC(O)CH₃, -CH(OH)CH₃ ou -C(O)CH₃.

6. Procédé selon la revendication 4 ou 5 **caractérisé en ce que** R" est un groupe -CH(OH)CH₃ ou -C(O)CH₃.

7. Procédé selon la revendication 1 ou 2 **caractérisé en ce qu'**il est mis en oeuvre pour la préparation d'un composé de formule (IA) : 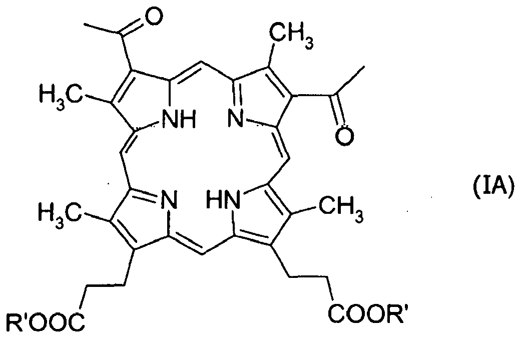 dans laquelle R' est tel que défini pour (I), ou un de ses sels, par couplage du pyrrométhane de formule (II) : 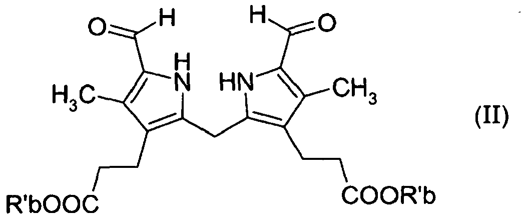 dans laquelle R'b est tel que défini précédemment pour (I),
et un dipyrrométhane de formule (IIIa) : 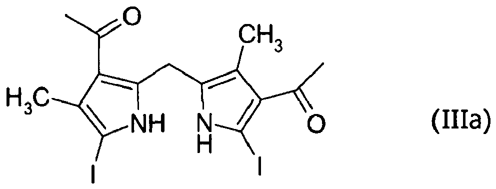 suivi, dans le cas où R' représente un atome d'hydrogène, d'une étape d'élimination de R'b par une réaction d'hydrolyse pour obtenir les groupes -COOH,
ou, éventuellement dans le cas où le composé (IA) à former est sous la forme d'un sel, d'une étape de saponification.

8. Procédé selon la revendication 7 **caractérisé en ce que** R'b est un groupe méthyle.

9. Procédé selon la revendication 1 ou 2 **caractérisé en ce qu'**il est mis en oeuvre pour la préparation d'un composé de formule (IB) : 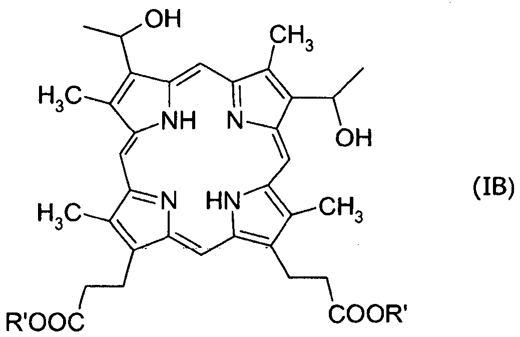 dans laquelle R' est tel que défini pour (I), ou un de ses sels,
par couplage du pyrrométhane de formule (II) : 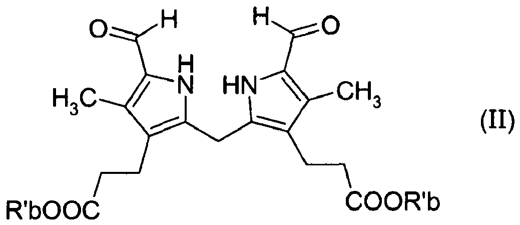 dans laquelle R'b est tel que défini précédemment pour (I),
et un dipyrrométhane de formule (IIIa) : 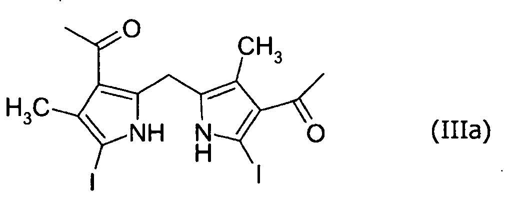 pour former le composé de formule (la) : 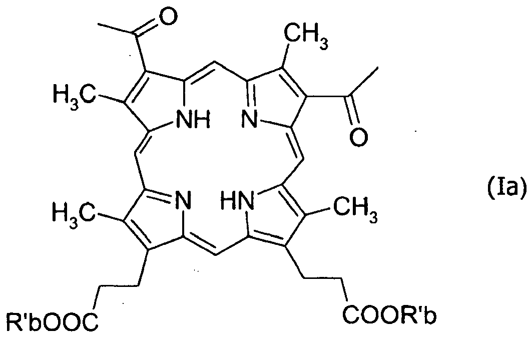 dans laquelle R'b est tel que défini pour (I)
suivi, d'une réduction des groupes -C(O)CH₃ en -CH(OH)CH₃,
et, dans le cas où R' représente un atome d'hydrogène, d'une étape d'élimination de R'b par une réaction d'hydrolyse pour obtenir les groupes -COOH,
ou bien, dans le cas où le composé (IB) à former est sous la forme d'un sel, d'une étape de saponification.

10. Procédé selon la revendication 9 **caractérisé en ce que** la réduction des groupes - C(O)CH₃ est réalisée en présence d'un hydrure.

11. Procédé selon la revendication 9 ou 10 **caractérisé en ce que** la réduction des groupes -C(O)CH₃ est réalisée dans le dichlorométhane en présence de méthanol.

12. Procédé selon l'une des revendications 1, 2 et 4 à 6 **caractérisé en ce qu'**il est mis en oeuvre pour la préparation du composé de formule (IC) : 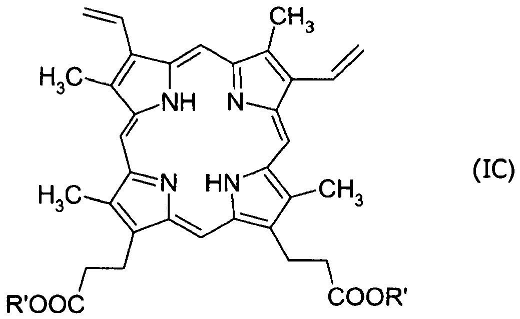 dans laquelle R' est tel que défini pour (I), ou un de ses sels,
par couplage du pyrrométhane de formule (II) : 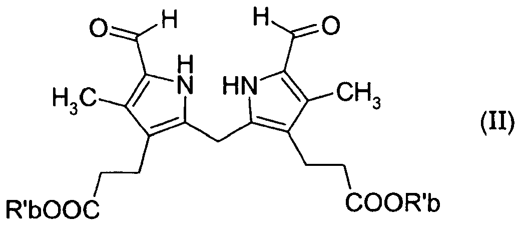 dans laquelle R'b est tel que défini précédemment pour (I),
et un dipyrrométhane de formule (IIIa) : 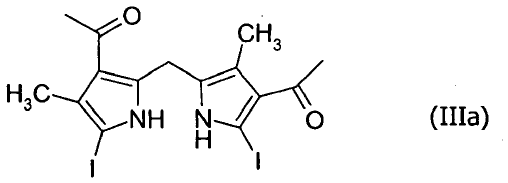 pour former le composé de formule (Ia) : 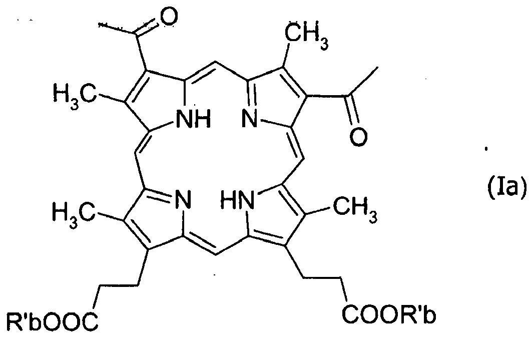 dans laquelle R'b est tel que défini pour (I),
suivi :
- d'une réduction des groupes -C(O)CH₃, conduisant à la formation de la porphyrine de formule (Ib) : 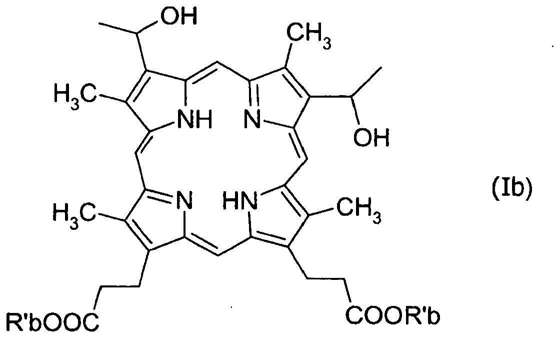
dans laquelle R'b est tel que défini pour (I),
- suivi d'une réaction d'élimination transformant les groupes -CH(OH)CH₃ en -CH=CH₂,
- et, dans le cas où R' représente un atome d'hydrogène, d'une étape d'élimination de R'b par hydrolyse pour obtenir les groupes -COOH,
ou, dans le cas où le composé (IC) à former est sous la forme d'un sel, d'une étape de saponification.

13. Procédé selon la revendication 12 **caractérisé en ce que** la réaction d'élimination transformant les groupes -CH(OH)CH₃ en -CH=CH₂ est réalisée en présence d'un halogénure d'acide.

14. Procédé selon la revendication 12 ou 13 **caractérisé en ce que** la réaction d'élimination transformant les groupes -CH(OH)CH₃ en -CH=CH₂, est réalisée dans un solvant polaire aprotique.

15. Procédé selon l'une des revendications 12 à 14 **caractérisé en ce que** la réduction des groupes -C(O)CH₃ est réalisée en présence d'un hydrure.

16. Procédé selon l'une des revendications 12 à 15 **caractérisé en ce que** la réduction des groupes -C(O)CH₃ est réalisée dans le dichlorométhane en présence de méthanol.

17. Procédé selon l'une des revendications 1 à 16 **caractérisé en ce que** R'b est un groupe méthyle.

18. Procédé selon l'une des revendications 12 à 17 de préparation de la protoporphyrine (IX) sous la forme du sel de sodium de formule (IC.2,2Na) : 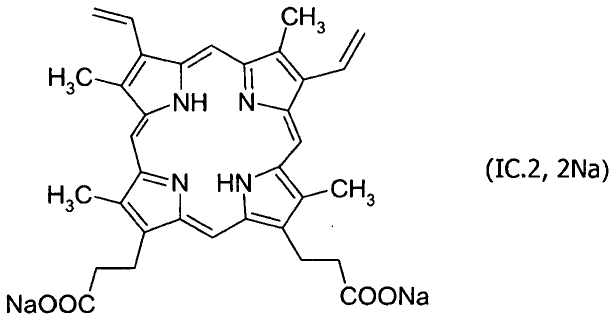 dans lequel une étape de saponification est réalisée sur le composé de formule (Ic) : 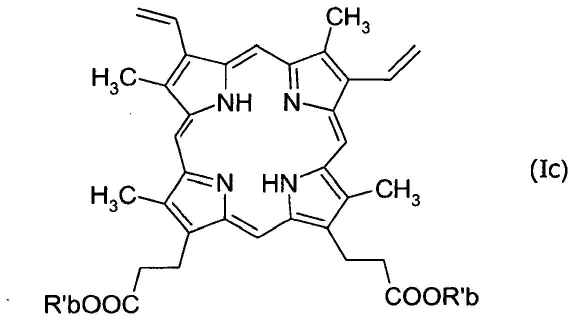 dans laquelle R'b est telle que définie à la revendication 1, par action de soude en présence de méthanol.

19. Procédé selon la revendication 18 **caractérisé en ce que** l'étape de saponification est réalisée dans le dichlorométhane à reflux.

20. Procédé selon l'une des revendications précédentes **caractérisé en ce que** la réaction de condensation entre les deux pyrrométhanes (II) et (III) ou (IIIa) est réalisée en présence d'un acide choisi parmi les acides carboxyliques, l'acide trifluoroacétique, l'acide chlorhydrique, l'acide trichlorométhanesulfonique, l'acide méthanesulfonique, l'acide trifluorométhanesulfonique, l'acide tetrafluoroborique, l'acide bromhydrique, l'acide iodhydrique.

21. Procédé selon la revendication 20 **caractérisé en ce que** l'acide est l'acide trifluoroacétique ou l'acide trichlorométhanesulfonique.

22. Procédé selon l'une des revendications 1 à 21 **caractérisé en ce que** la réaction de condensation entre les deux pyrrométhanes (II) et (III) ou (IIIa) est réalisée en présence d'un agent dessiccateur.

23. Procédé selon la revendication 22 **caractérisé en ce que** l'agent dessiccateur est choisi parmi les anhydrides, les tamis moléculaires, l'acide sulfurique.

24. Procédé selon la revendication 23 **caractérisé en ce que** l'agent dessiccateur est l'anhydride acétique.

25. Procédé selon la revendication 24 **caractérisé en ce que** l'anhydride acétique est présent en excès, de préférence, d'au moins 10 équivalents molaires par rapport au pyrrométhane (II).

26. Procédé selon l'une des revendications 1 à 25 **caractérisé en ce que** la réaction de condensation entre les deux pyrrométhanes (II) et (III) ou (IIIa) est réalisée selon un ratio en équivalent molaire compris entre 1 et 1,2.

27. Procédé selon l'une des revendications 1 à 26 **caractérisé en ce que** la réaction de condensation entre les deux pyrrométhanes (II) et (III) ou (IIIa) est réalisée à une température de 10 à 50°C dans un solvant protique.

28. Procédé de préparation d'un complexe métallique d'une porphyrine de formule (I), éventuellement sous la forme d'un sel : 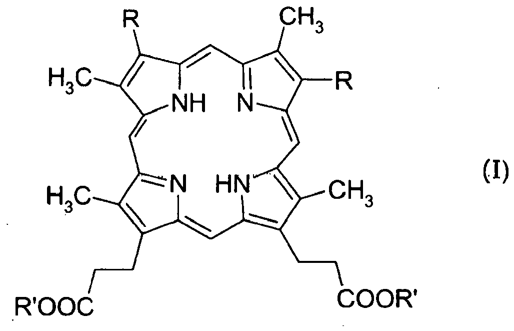 dans laquelle :
- R représente un atome d'hydrogène ou un groupe choisi parmi : -CH=CH₂, -CH₂-CH₃, - CH(OH)CH₃, -C(O)CH₃, -CH₂CH₂COOR'a avec R'a qui représente un atome d'hydrogène ou un groupe méthyle, éthyle, n-propyle ou i-propyle,
- R' représente un atome d'hydrogène ou un groupe R'b choisi parmi méthyle, éthyle, n-propyle ou i-propyle,
le procédé comprenant une étape de complexation d'une porphyrine de formule (I) formée après l'étape de condensation entre les composés (II) et (III) ou (IIIa) avec un métal, un sel de métal ou un hydroxyde de métal.

29. Un procédé de préparation d'un complexe métallique selon la revendication 28 selon laquelle le métal, le composant métallique du sel de métal, ou le composant métallique - de l'hydroxyde de métal est choisi parmi le fer, le galium, le nickel, le zinc, le palladium, le cobalt, le calcium ou le magnésium.

30. Procédé selon la revendication 29 **caractérisé en ce que** l'étape de complexation est réalisée, en étape finale, sur la porphyrine de formule (I), éventuellement sous la forme d'un sel.

31. Procédé selon la revendication 29 ou 30 **caractérisé en ce qu'**il est mis en oeuvre pour la formation de l'hème, l'hémine ou l'hématine.

32. Pyrrométhanes de formule (III) : 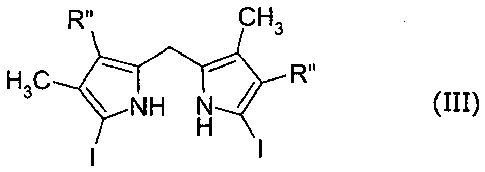 dans laquelle R" est un atome d'hydrogène ou un groupe choisi parmi : -CH=CH₂, -CH₂-CH₃, - CH(OH)CH₃, -C(O)CH₃, -CH₂CH₂COOR'a avec R'a qui représente un atome d'hydrogène ou un groupe méthyle, éthyle, n-propyle ou i-propyle.

33. Pyrrométhane selon la revendication 32 dans lequel R" est un groupe précurseur de -CH=CH₂ choisi parmi : -CH₂CH₂OC(O)CH₃, -CH₂CH₂OH, -CH₂CH₂Cl, -CH(OH)CH₃, - C(O)CH₃.

34. Pyrrométhane selon la revendication 32 ou 33 dans lequel R" est un groupe -CH(OH)CH₃ ou -C(O)CH₃.

35. Composés choisis parmi les composés de formule (VIIa), (Va) et (IVa) :

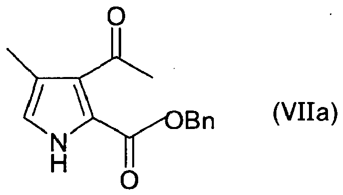

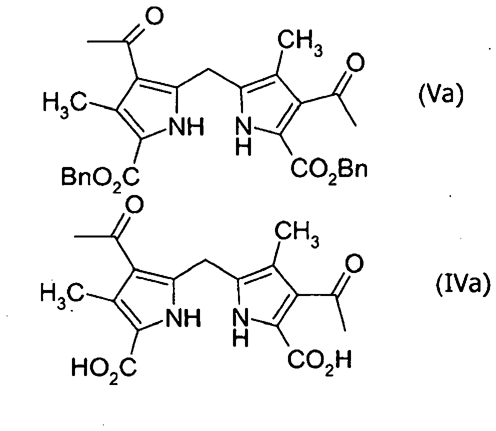

## Patentansprüche

1. Verfahren zur Herstellung eines Porphyrins der Formel (I), gegebenenfalls in Form eines Salzes: 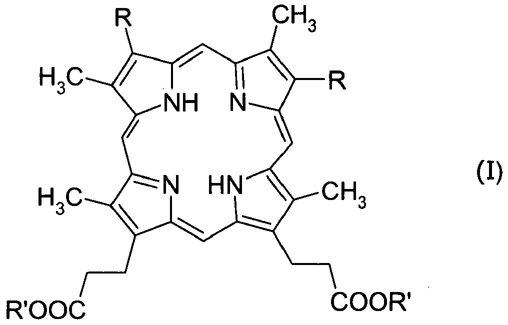 worin:
- R für ein Wasserstoffatom oder eine aus -CH=CH₂,
- CH₂-CH₃, -CH (OH) CH3, -C(O)CH₃ und -CH₂CH₂COOR'a ausgewählte Gruppe steht, wobei R'a für ein Wasserstoffatom oder eine Methyl-, Ethyl-, n-Propyl- oder i-Propylgruppe steht,
- R' für ein Wasserstoffatom oder eine Gruppe R'b, die aus Methyl, Ethyl, n-Propyl oder i-Propyl ausgewählt ist, steht,
umfassend:
- einen Schritt der Kondensation zwischen einem Dipyrromethan der Formel (II): 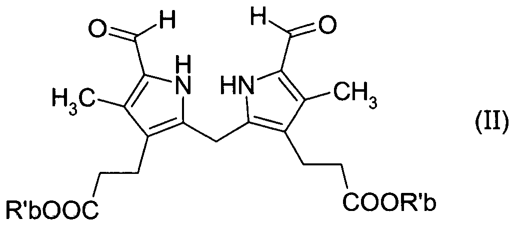
wobei R'b wie oben für (I) definiert ist, und einem Dipyrromethan der Formel (III): 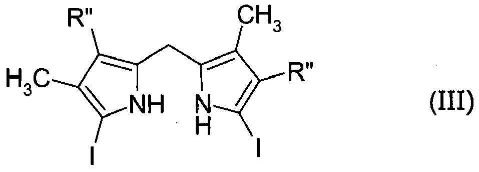 worin R'' mit R wie oben für (I) definiert identisch ist oder eine Vorläufergruppe von R ist, in saurem Medium zu dem Porphyrin der Formel (I'): 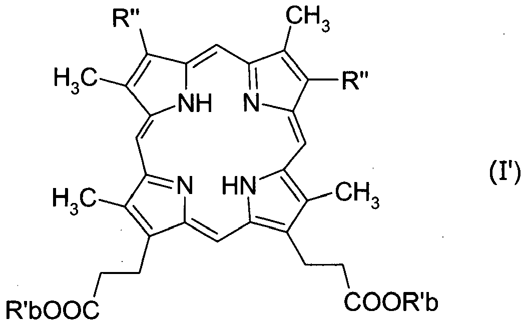 worin R" und R'b wie oben für (II) und (III) definiert sind,
gefolgt von:
- wenn R'' eine Vorläufergruppe von R ist, der Umwandlung der Gruppen R" in R,
- wenn R' = H, einer Eliminierung der Gruppen R'b zur Bildung von -COOH-Gruppen, gegebenenfalls in Salzform.

2. Verfahren nach Anspruch 1, bei dem R'' ein Wasserstoffatom oder eine aus -CH=CH₂, -CH₂-CH₃, -CH(OH) CH₃, -C(O)CH₃, -CH₂CH₂OH, -CH₂CH₂Cl, -CH₂CH₂OC(O)CH₃ und -CH₂CH₂COOR'a ausgewählte Gruppe ist, wobei R'a für ein Wasserstoffatom oder eine Methyl-, Ethyl-, n-Propyl- oder i-Propylgruppe steht.

3. Verfahren nach Anspruch 1 oder 2, **dadurch gekennzeichnet, dass** es zur Herstellung einer Verbindung der Formel (I), worin R für ein Wasserstoffatom oder eine aus -CH₂-CH₃, -CH(OH)CH₃, -C(O)CH₃ und -CH₂CH₂COOR'a ausgewählte Gruppe steht, wobei R'a für ein Wasserstoffatom oder eine Methyl-, Ethyl-, n-Propyl- oder i-Propylgruppe steht, eingesetzt wird.

4. Verfahren nach Anspruch 1 oder 2, **dadurch gekennzeichnet, dass** es zur Herstellung einer Verbindung der Formel (I), worin R für eine -CH=CH₂-Gruppe steht, eingesetzt wird, umfassend:
- einen Schritt der Kondensation zwischen einem Dipyrromethan der Formel (II): 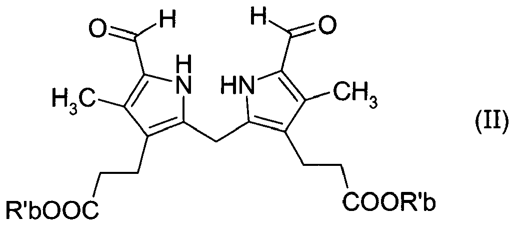 worin R'b wie oben für (I) definiert ist, und einem Dipyrromethan der Formel (III): 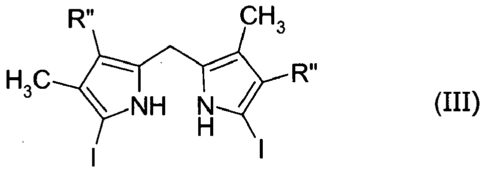 worin R'' eine Vorläufergruppe von R ist, in saurem Medium gefolgt von der Umwandlung der Gruppen R'' in R,
und wenn R' = H, der Eliminierung der Gruppen R'b zum Erhalt von -COOH-Gruppen, gegebenenfalls in Salzform.

5. Verfahren nach Anspruch 4, **dadurch gekennzeichnet, dass** R'' eine -CH₂CH₂OH-, -CH₂CH₂Cl-, -CH₂CH₂OC(O)CH₃-, -CH(OH)CH₃- oder -C(O)CH₃-Gruppe ist.

6. Verfahren nach Anspruch 4 oder 5, **dadurch gekennzeichnet, dass** R'' eine -CH(OH)CH₃- oder -C(O)CH₃-Gruppe ist.

7. Verfahren nach Anspruch 1 oder 2, **dadurch gekennzeichnet, dass** es zur Herstellung einer Verbindung der Formel (IA): 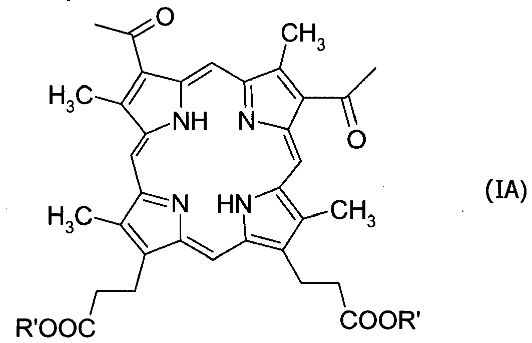 worin R' wie für (I) definiert ist, oder eines ihrer Salze durch Kupplung des Pyrromethans der Formel (II): 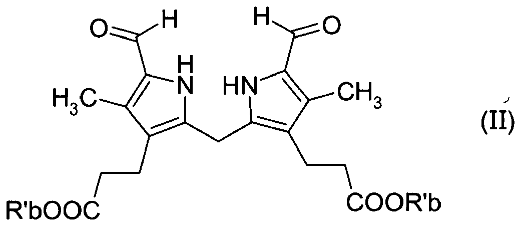 worin R'b wie oben für (I) definiert ist, und eines Dipyrromethans der Formel (IIIa): 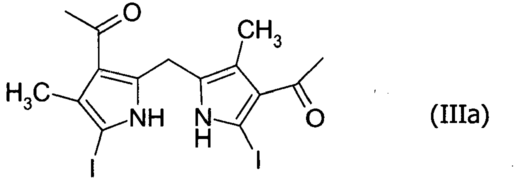 in dem Fall, dass R' für ein Wasserstoffatom steht, gefolgt von einem Schritt der Eliminierung von R'b durch eine Hydrolysereaktion zum Erhalt von -COOH-Gruppen
oder gegebenenfalls in dem Fall, dass die zu bildende Verbindung (IA) in Form eines Salzes vorliegt, gefolgt von einem Verseifungsschritt, eingesetzt wird.

8. Verfahren nach Anspruch 7, **dadurch gekennzeichnet, dass** R'b eine Methylgruppe ist.

9. Verfahren nach Anspruch 1 oder 2, **dadurch gekennzeichnet, dass** es zur Herstellung einer Verbindung der Formel (IB): 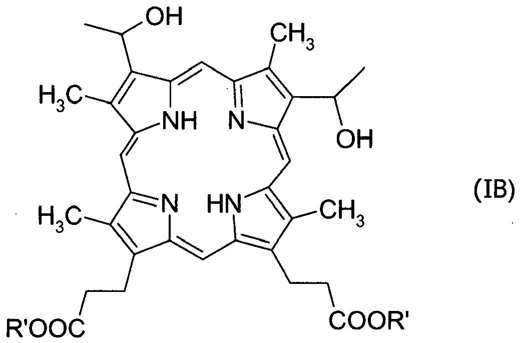 worin R' wie für (I) definiert ist, oder eines ihrer Salze
durch Kupplung des Pyrromethans der Formel (II): 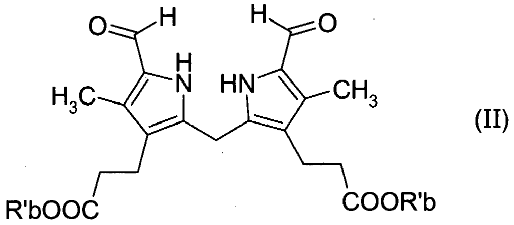 worin R'b wie oben für (I) definiert ist, und eines Dipyrromethans der Formel (IIIa): 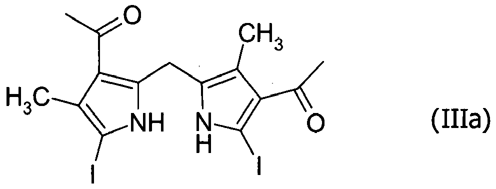 zu der Verbindung der Formel (Ia): 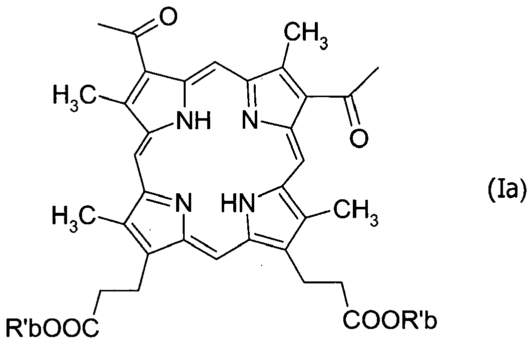 worin R'b wie für (I) definiert ist,
gefolgt von einer Reduktion der -C(O)CH₃-Gruppen zu -CH(OH)CH₃,
und in dem Fall, dass R' für ein Wasserstoffatom steht, von einem Schritt der Eliminierung von R'b durch eine Hydrolysereaktion zum Erhalt von -COOH-Gruppen
oder auch in dem Fall, dass die zu bildende Verbindung (IB) in Form eines Salzes vorliegt, von einem Verseifungsschritt,
eingesetzt wird.

10. Verfahren nach Anspruch 9, **dadurch gekennzeichnet, dass** die Reduktion der -C(O)CH₃-Gruppen in Gegenwart eines Hydrids durchgeführt wird.

11. Verfahren nach Anspruch 9 oder 10, **dadurch gekennzeichnet, dass** die Reduktion der -C(O)CH₃-Gruppen in Dichlormethan in Gegenwart von Methanol durchgeführt wird.

12. Verfahren nach einem der Ansprüche 1, 2 und 4 bis 6, **dadurch gekennzeichnet, dass** es zur Herstellung der Verbindung der Formel (IC) 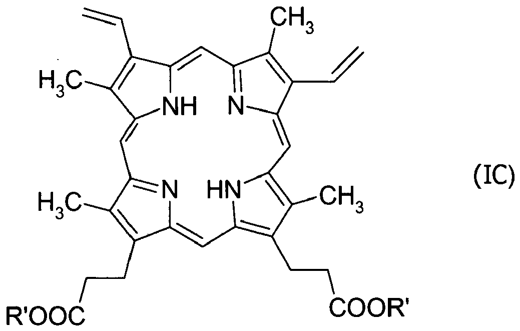 worin R' wie für (I) definiert ist, oder eines ihrer Salze
durch Kupplung des Pyrromethans der Formel (II): 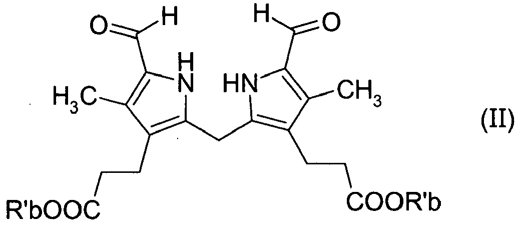 worin R'b wie oben für (I) definiert ist,
und eines Dipyrromethans der Formel (IIIa): 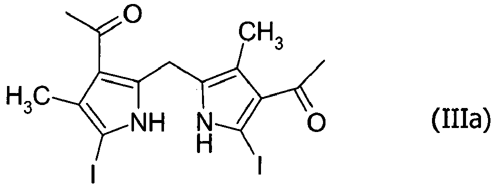 zu der Verbindung der Formel (Ia): 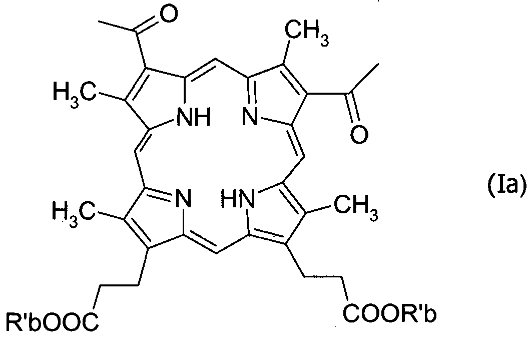 worin R'b wie für (I) definiert ist,
gefolgt von
- einer Reduktion der -C(O)CH₃-Gruppen, was zur Bildung des Porphyrins der Formel (Ib) führt: 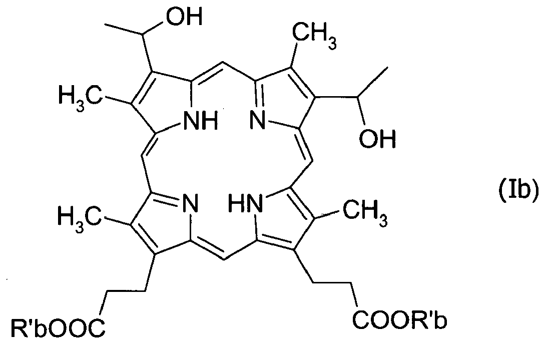
worin R'b wie für (I) definiert ist,
gefolgt von einer Eliminierungsreaktion zur Umwandlung der -CH(OH)CH₃-Gruppen in -CH=CH₂,
und in dem Fall, dass R' für ein Wasserstoffatom steht, von einem Schritt der Eliminierung von R'b durch Hydrolyse zum Erhalt von -COOH-Gruppen oder in dem Fall, dass die zu bildende Verbindung (IC) in Form eines Salzes vorliegt, von einem Verseifungsschritt,
eingesetzt wird.

13. Verfahren nach Anspruch 12, **dadurch gekennzeichnet, dass** die Eliminierungsreaktion zur Umwandlung der -CH(OH)CH₃-Gruppen in -CH=CH₂, in Gegenwart eines Säurehalogenids durchgeführt wird.

14. Verfahren nach Anspruch 12 oder 13, **dadurch gekennzeichnet, dass** die Eliminierungsreaktion zur Umwandlung der -CH(OH)CH₃-Gruppen in -CH=CH₂, in einem polaren aprotischen Lösungsmittel durchgeführt wird.

15. Verfahren nach einem der Ansprüche 12 bis 14, **dadurch gekennzeichnet, dass** die Reduktion der -C(O)CH₃-Gruppen in Gegenwart eines Hydrids durchgeführt wird.

16. Verfahren nach einem der Ansprüche 12 bis 15, **dadurch gekennzeichnet, dass** die Reduktion der -C(O)CH₃-Gruppen in Dichlormethan in Gegenwart von Methanol durchgeführt wird.

17. Verfahren nach einem der Ansprüche 1 bis 16, **dadurch gekennzeichnet, dass** R'b eine Methylgruppe ist.

18. Verfahren nach einem der Ansprüche 12 bis 17 zur Herstellung des Protoporphyrins (IX) in Form des Natriumsalzes der Formel (IC.2,2Na): 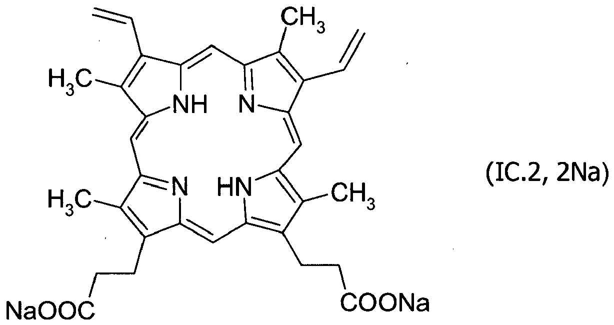 bei dem man die Verbindung der Formel (Ic): 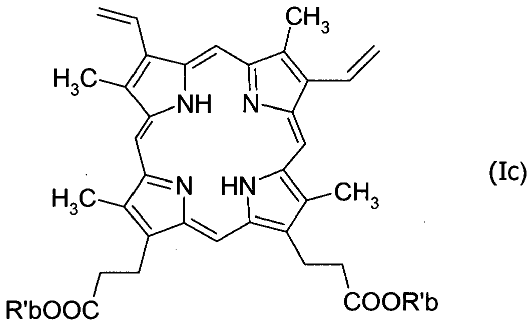 worin R'b wie in Anspruch 1 definiert ist, durch Einwirkung von Natriumhydroxid in Gegenwart von Methanol verseift.

19. Verfahren nach Anspruch 18, **dadurch gekennzeichnet, dass** man den Verseifungsschritt in Dichlormethan unter Rückfluss durchführt.

20. Verfahren nach einem der vorhergehenden Ansprüche, **dadurch gekennzeichnet, dass** man die Kondensationsreaktion zwischen den beiden Pyrromethanen (II) und (III) bzw. (IIIa) in Gegenwart einer aus Carbonsäuren, Trifluoressigsäure, Salzsäure, Trichlormethansulfonsäure, Methansulfonsäure, Trifluormethansulfonsäure, Tetrafluoroborsäure, Bromwasserstoffsäure und Iodwasserstoffsäure ausgewählten Säure durchführt.

21. Verfahren nach Anspruch 20, **dadurch gekennzeichnet, dass** es sich bei der Säure um Trifluoressigsäure oder Trichlormethansulfonsäure handelt.

22. Verfahren nach einem der Ansprüche 1 bis 21, **dadurch gekennzeichnet, dass** man die Kondensationsreaktion zwischen den beiden Pyrromethanen (II) und (III) bzw. (IIIa) in Gegenwart eines Trockenmittels durchführt.

23. Verfahren nach Anspruch 22, **dadurch gekennzeichnet, dass** das Trockenmittel aus Anhydriden, Molsieben und Schwefelsäure ausgewählt wird.

24. Verfahren nach Anspruch 23, **dadurch gekennzeichnet, dass** es sich bei dem Trockenmittel um Essigsäureanhydrid handelt.

25. Verfahren nach Anspruch 24, **dadurch gekennzeichnet, dass** das Essigsäureanhydrid im Überschuss, vorzugsweise von mindestens 10 Moläquivalenten, bezogen auf das Pyrromethan (II), vorliegt.

26. Verfahren nach einem der Ansprüche 1 bis 25, **dadurch gekennzeichnet, dass** man die Kondensationsreaktion zwischen den beiden Pyrromethanen (II) und (III) bzw. (IIIa) gemäß einem Moläquivalentverhältnis zwischen 1 und 1,2 durchführt.

27. Verfahren nach einem der Ansprüche 1 bis 26, **dadurch gekennzeichnet, dass** man die Kondensationsreaktion zwischen den beiden Pyrromethanen (II) und (III) bzw. (IIIa) bei einer Temperatur von 10 bis 50°C in einem protischen Lösungsmittel durchführt.

28. Verfahren zur Herstellung eines Metallkomplexes eines Porphyrins der Formel (I), gegebenenfalls in Form eines Salzes: 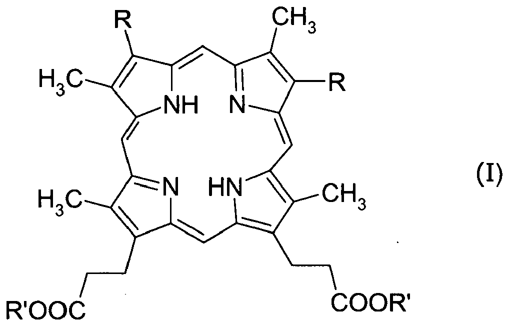 worin:
- R für ein Wasserstoffatom oder eine aus -CH=CH₂, -CH₂-CH₃, -CH(OH)CH₃, -C(O)CH₃ und -CH₂CH₂COOR'a ausgewählte Gruppe steht, wobei R'a für ein Wasserstoffatom oder eine Methyl-, Ethyl-, n-Propyl- oder i-Propylgruppe steht,
- R' für ein Wasserstoffatom oder eine Gruppe R'b, die aus Methyl, Ethyl, n-Propyl oder i-Propyl ausgewählt ist, steht,
wobei das Verfahren einen Schritt der Komplexierung eines nach dem Schritt der Kondensation zwischen den Verbindungen (II) und (III) bzw. (IIIa) gebildeten Porphyrins der Formel (I) mit einem Metall, einem Metallsalz oder einem Metallhydroxid umfasst.

29. Verfahren zur Herstellung eines Metallkomplexes nach Anspruch 28, gemäß dem das Metall, die Metallkomponente des Metallsalzes oder die Metallkomponente des Metallhydroxids aus Eisen, Gallium, Nickel, Zink, Palladium, Cobalt, Calcium oder Magnesium ausgewählt wird.

30. Verfahren nach Anspruch 29, **dadurch gekennzeichnet, dass** der Komplexierungsschritt als letzter Schritt an dem Porphyrin der Formel (I), gegebenenfalls in Form eines Salzes, durchgeführt wird.

31. Verfahren nach Anspruch 29 oder 30, **dadurch gekennzeichnet, dass** es zur Bildung von Häm, Hämin oder Hämatin eingesetzt wird.

32. Pyrromethane der Formel (III): 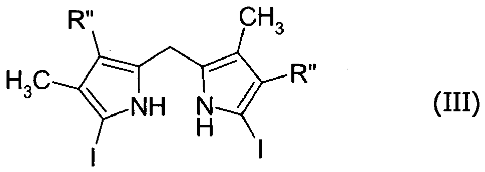 worin R'' für ein Wasserstoffatom oder eine aus -CH=CH₂, -CH₂-CH₃, -CH(OH)CH₃, -C(O)CH₃ und -CH₂CH₂COOR'a ausgewählte Gruppe steht, wobei R'a für ein Wasserstoffatom oder eine Methyl-, Ethyl-, n-Propyl- oder i-Propylgruppe steht.

33. Pyrromethan nach Anspruch 32, worin R'' eine Vorläufergruppe von -CH=CH₂ ist, die aus -CH₂CH₂OC(O)CH₃, -CH₂CH₂OH, -CH₂CH₂Cl, -CH(OH)CH₃ und -C(O)CH₃ ausgewählt ist.

34. Pyrromethan nach Anspruch 32 oder 33, worin R'' eine -CH(OH)CH₃- oder -C(O)CH₃-Gruppe ist.

35. Verbindungen, ausgewählt aus den Verbindungen der Formel (VIIa), (Va) und (IVa):

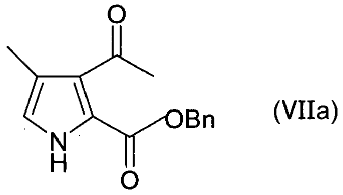

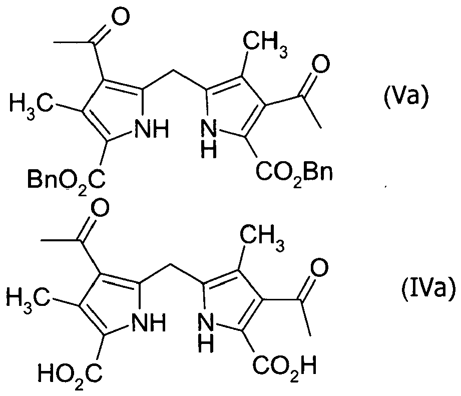

## Claims

1. Process for preparing a porphyrin of formula (I), optionally in the form of a salt: 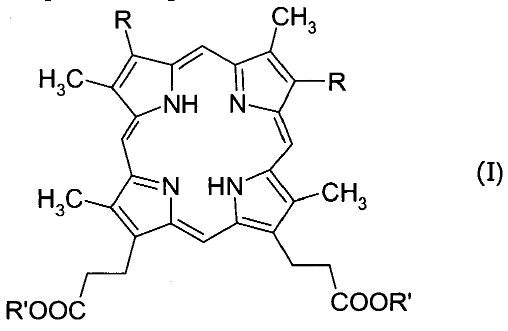 in which:
- R is a hydrogen atom or a group selected from: - CH=CH₂, -CH₂-CH₃, -CH (OH) CH3, -C(O)CH₃ and -CH₂CH₂COOR'a, with R'a being a hydrogen atom or a methyl, ethyl, n-propyl or i-propyl group,
- R' is a hydrogen atom or a group R'b selected from methyl, ethyl, n-propyl or i-propyl,
comprising:
- a step of condensation, in an acidic medium, between a dipyrromethane of formula (II): 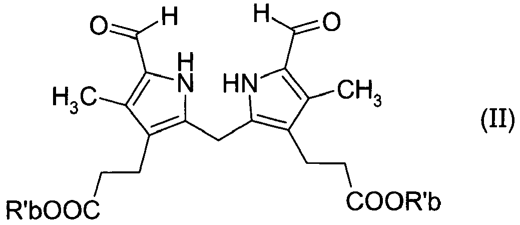
in which R'b is as defined above for (I),
and a dipyrromethane of formula (III): 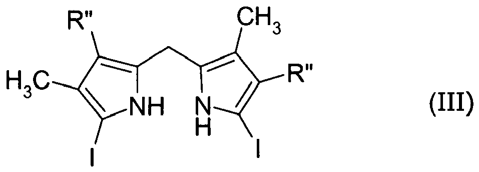 in which R'' is identical to R as defined above for (I) or is a group that is a precursor of R, so as to form the porphyrin of formula (I'): 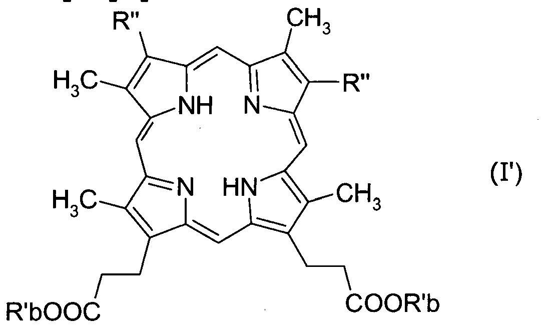 in which R'' and R'b are as defined above for (II) and (III),
followed:
- when **R**'' is a group that is a precursor of R, by conversion of the groups R'' to R,
- when R'=H, by elimination of the groups R'b so as to form -COOH groups, optionally in the form of a salt.

2. Process according to Claim 1, in which R'' is a hydrogen atom or a group selected from: -CH=CH₂, -CH₂-CH₃, -CH(OH)CH₃, -C(O)CH₃, -CH₂CH₂OH, -CH₂CH₂Cl and -CH₂CH₂OC(O)CH₃ and -CH₂CH₂COOR'a, with R'a being a hydrogen atom or a methyl, ethyl, n-propyl or i-propyl group.

3. Process according to Claim 1 or 2, **characterized in that** it is implemented for the preparation of a compound of formula (I) in which R is a hydrogen atom or a group selected from: -CH₂-CH₃, -CH(OH)CH₃, -C(O)CH₃ and -CH₂CH₂COOR'a, with R'a being a hydrogen atom or a methyl, ethyl, n-propyl or i-propyl group.

4. Process according to Claim 1 or 2, **characterized in that** it is implemented for the preparation of a compound of formula (I) in which R is a -CH=CH₂ group, comprising:
a step of condensation, in an acidic medium, between a dipyrromethane of formula (II): 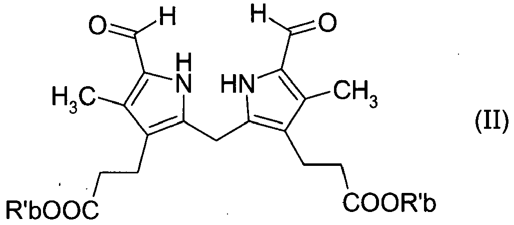 in which R'b is as defined above for (I),
and a dipyrromethane of formula (III): 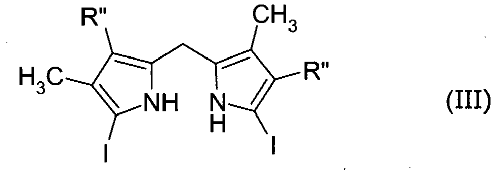
in which R'' is a group that is a precursor of R, followed by conversion of the groups R'' to R,
and when R'=H, by elimination of the groups R'b so as to obtain -COOH groups, optionally in the form of a salt.

5. Process according to Claim 4, **characterized in that** R'' is a -CH₂CH₂OH, -CH₂CH₂Cl, -CH₂CH₂OC(O)CH₃, -CH(OH)CH₃ or - C(O)CH₃ group.

6. Process according to Claim 4 or 5, **characterized in that** R'' is a -CH(OH)CH₃ or -C(O)CH₃ group.

7. Process according to Claim 1 or 2, **characterized in that** it is implemented for the preparation of a compound of formula (IA): 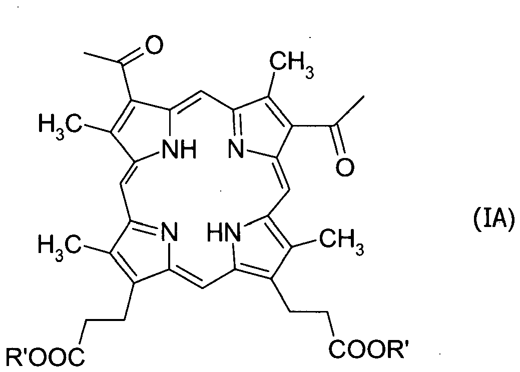 in which R' is as defined for (I), or a salt thereof, by coupling the pyrromethane of formula (II): 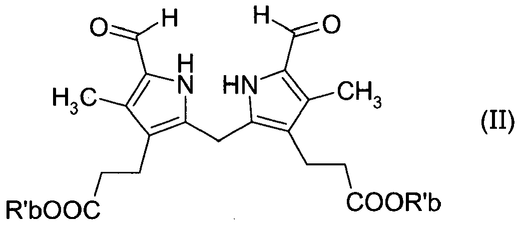 in which R'b is as defined above for (I), and a dipyrromethane of formula (IIIa): 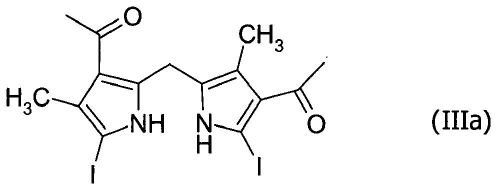 followed, in the case where R' is a hydrogen atom, by a step of eliminating R'b by a hydrolysis reaction so as to obtain -COOH groups,
or, optionally in the case where the compound (IA) to be formed is in the form of a salt, by a saponification step.

8. Process according to Claim 7, **characterized in that** R'b is a methyl group.

9. Process according to Claim 1 or 2, **characterized in that** it is implemented for the preparation of a compound of formula (IB): 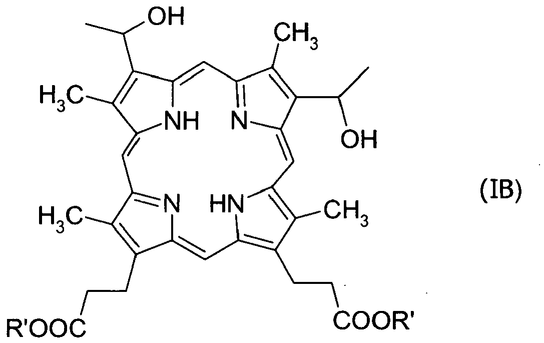 in which R' is as defined for (I), or a salt thereof, by coupling the pyrromethane of formula (II): 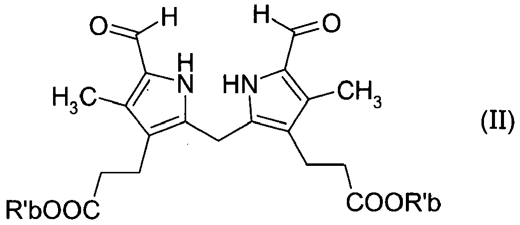 in which R'b is as defined above for (I), and a dipyrromethane of formula (IIIa): 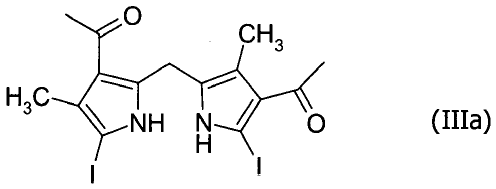 so as to form the compound of formula (Ia): 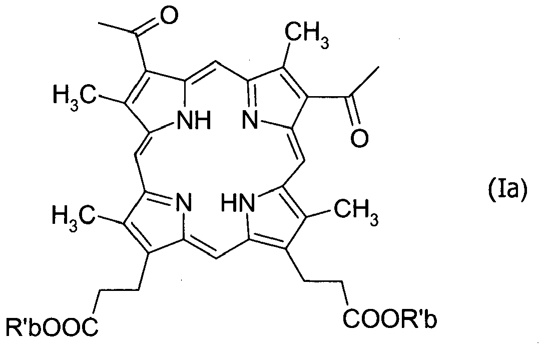 in which R'b is as defined for (I),
followed by reduction of the -C(O)CH₃ groups to - CH(OH)CH₃,
and, in the case where R' is a hydrogen atom, by a step of eliminating R'b by a hydrolysis reaction so as to obtain -COOH groups,
or else, in the case where the compound (IB) to be formed is in the form of a salt, by a saponification step.

10. Process according to Claim 9, **characterized in that** the reduction of the -C(O)CH₃ groups is carried out in the presence of a hydride.

11. Process according to Claim 9 or 10, **characterized in that** the reduction of the -C(O)CH₃ groups is carried out in dichloromethane in the presence of methanol.

12. Process according to one of Claims 1, 2 and 4 to 6, **characterized in that** it is implemented for the preparation of the compound of formula (IC): 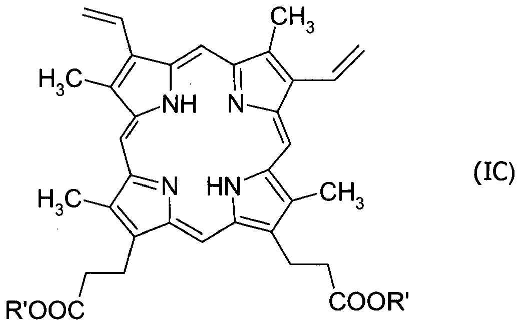 in which R' is as defined for (I), or a salt thereof,
by coupling the pyrromethane of formula (II): 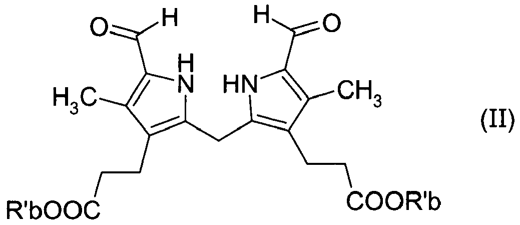 in which R'b is as defined above for (I),
and a dipyrromethane of formula (IIIa): 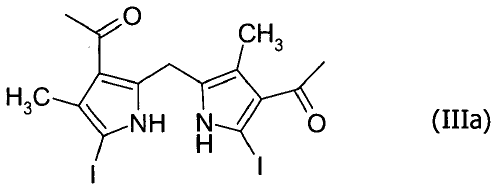 so as to form the compound of formula (Ia): 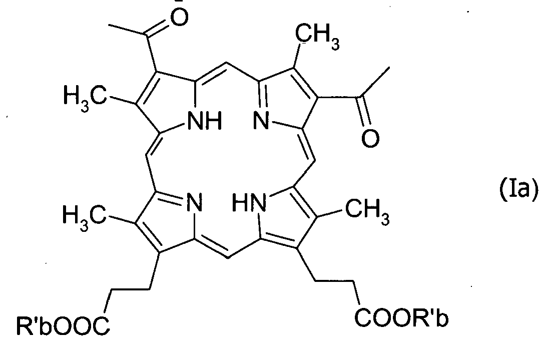 in which R'b is as defined for (I),
followed:
- by reduction of the -C(O)CH₃ groups, resulting in the formation of the porphyrin of formula (Ib): 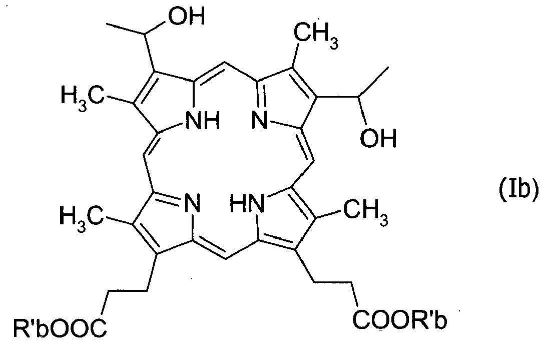
in which R'b is as defined for (I),
- followed by an elimination reaction that converts the groups -CH(OH)CH₃ to -CH=CH₂,
- and, in the case where R' is a hydrogen atom, by a step of eliminating R'b by hydrolysis so as to obtain - COOH groups,
or, in the case where the compound (IC) to be formed is in the form of a salt, by a saponification step.

13. Process according to Claim 12, **characterized in that** the elimination reaction that converts the groups -CH(OH)CH₃ to -CH=CH₂ is carried out in the presence of an acid halide.

14. Process according to Claim 12 or 13, **characterized in that** the elimination reaction that converts the groups -CH(OH)CH₃ to -CH=CH₂ is carried out in an aprotic polar solvent.

15. Process according to one of Claims 12 to 14, **characterized in that** the reduction of the -C(O)CH₃ groups is carried out in the presence of a hydride.

16. Process according to one of Claims 12 to 15, **characterized in that** the reduction of the -C(O)CH₃ groups is carried out in dichloromethane in the presence of methanol.

17. Process according to one of Claims 1 to 16, **characterized in that** R'b is a methyl group.

18. Process according to one of Claims 12 to 17 for preparing protoporphyrin (IX) in the form of the sodium salt of formula (IC.2, 2Na): 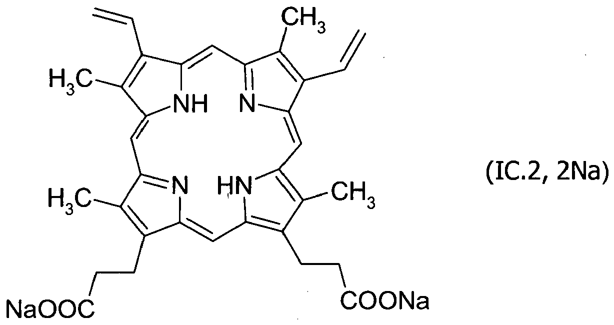 in which a saponification step is carried out on the compound of formula (Ic): 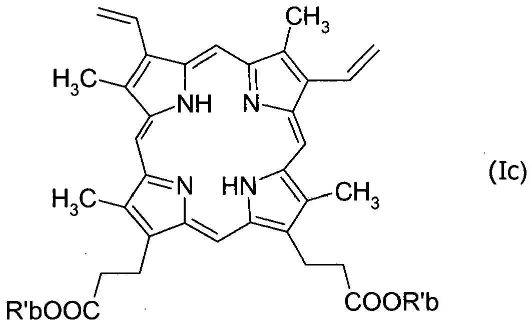 in which R'b is as defined in Claim 1, by the action of sodium hydroxide in the presence of methanol.

19. Process according to Claim 18, **characterized in that** the saponification step is carried out in dichloromethane at reflux.

20. Process according to one of the preceding claims, **characterized in that** the condensation reaction between the two pyrromethanes (II) and (III) or (IIIa) is carried out in the presence of an acid selected from the carboxylic acids, trifluoroacetic acid, hydrochloric acid, trichloromethanesulfonic acid, methanesulfonic acid, trifluoromethanesulfonic acid, tetrafluoroboric acid, hydrobromic acid and hydriodic acid.

21. Process according to Claim 20, **characterized in that** the acid is trifluoroacetic acid or trichloromethanesulfonic acid.

22. Process according to one of Claims 1 to 21, **characterized in that** the condensation reaction between the two pyrromethanes (II) and (III) or (IIIa) is carried out in the presence of a desiccating agent.

23. Process according to Claim 22, **characterized in that** the desiccating agent is selected from anhydrides, molecular sieves and sulfuric acid.

24. Process according to Claim 23, **characterized in that** the desiccating agent is acetic anhydride.

25. Process according to Claim 24, **characterized in that** the acetic anhydride is present in an excess, preferably of at least 10 molar equivalents relative to the pyrromethane (II).

26. Process according to one of Claims 1 to 25, **characterized in that** the condensation reaction between the two pyrromethanes (II) and (III) or (IIIa) is carried out according to a molar equivalent ratio of between 1 and 1.2.

27. Process according to one of Claims 1 to 26, **characterized in that** the condensation reaction between the two pyrromethanes (II) and (III) or (IIIa) is carried out at a temperature of from 10 to 50°C in a protic solvent.

28. Process for preparing a metal complex of a porphyrin of formula (I), optionally in the form of a salt: 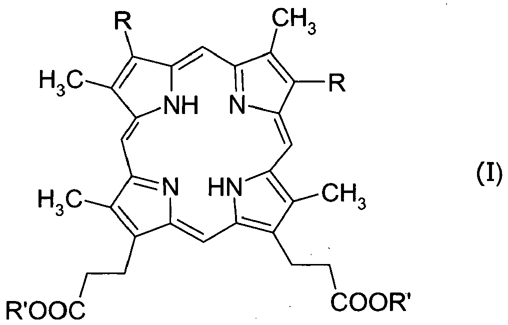 in which:
- R is a hydrogen atom or a group selected from: - CH=CH₂, -CH₂-CH₃, -CH(OH)CH₃, -C(O)CH₃ and -CH₂CH₂COOR'a, with R'a being a hydrogen atom or a methyl, ethyl, n-propyl or i-propyl group,
- R' is a hydrogen atom or a group R'b selected from methyl, ethyl, n-propyl or i-propyl,
the process comprising a step of complexation of a porphyrin of formula (I), formed after the step of condensation between the compounds (II) and (III) or (IIIa), with a metal, a metal salt or a metal hydroxide.

29. Process for preparing a metal complex according to Claim 28, according to which the metal, the metal component of the metal salt, or the metal component of the metal hydroxide is selected from iron, gallium, nickel, zinc, palladium, cobalt, calcium or magnesium.

30. Process according to Claim 29, **characterized in that** the complexation step is carried out, in a final step, on the porphyrin of formula (I), optionally in the form of a salt.

31. Process according to Claim 29 or 30, **characterized in that** it is implemented for the formation of heme, hemin or hematin.

32. Pyrromethanes of formula (III): 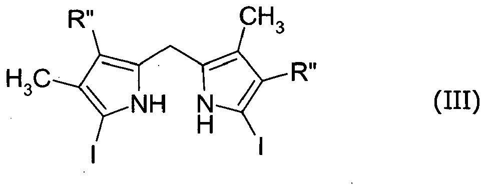 in which R'' is a hydrogen atom or a group selected from: -CH=CH₂, -CH₂-CH₃, -CH(OH)CH₃, -C(O)CH₃ and -CH₂CH₂COOR'a, with R'a being a hydrogen atom or a methyl, ethyl, n-propyl or i-propyl group.

33. Pyrromethane according to Claim 32, in which R'' is a group that is a precursor of -CH=CH₂, selected from: -CH₂CH₂OC(O)CH₃, -CH₂CH₂OH, -CH₂CH₂Cl, -CH(OH)CH₃ and -C(O)CH₃.

34. Pyrromethane according to Claim 32 or 33, in which R'' is a -CH(OH)CH₃ or -C(O)CH₃ group.

35. Compounds selected from the compounds of formulae (VIIa), (Va) and (IVa):
